# EUROPEAN PATENT APPLICATION

(11) **EP 4 704 099 A1**
(43) Date of publication of application: **04.03.2026**
(21) Application number: 25197094.3
(22) Date of filing: 20.08.2025
(51) Int. Cl.: G16H 20/30, A61C 13/00, G06N 3/02, G16H 30/40, G16H 50/50

(54) **SYSTEM AND METHOD FOR DENTAL RESTORATION USING NEURAL NETWORK**

(30) Priority: 30.08.2024 EP 24197549
(71) Applicant: 3Shape A/S, 1060 Copenhagen K (DK)
(72) Inventor: RASMUSSEN, Johannes Tophøj, 1060 Copenhagen K (DK); TRÄFF, Jens Peter, 1060 Copenhagen K (DK); SØNDERGAARD, Peter, 1060 Copenhagen K (DK)
(74) Representative: Zacco Denmark A/S

(57) **Abstract**

The present disclosure relates to computer-aided dental restoration system (102) that is configured to estimate crown pose and representing virtual crown in 3D model (110). The system obtains 3D model (110) of dentition of the patient. The system segments 3D model to obtain segmented tooth data and generates encoded 3D model representation suitable for processing by trained neural network (112). The method for generating encoded 3D model representation comprises subsampling point cloud (404) representation based on segmented tooth data, determining surface normal representation, retrieving dental notation of restorative site, encoding each of plurality of points with corresponding surface normal representation and with value relative to restorative site to produce encoded 3D model representation, inputting encoded 3D model representation into trained neural network, and producing output, using trained neural network, wherein output comprises prediction of translation of each point of encoded 3D model representation into positions corresponding to crown pose.

## Description

### TECHNICAL FIELD

The present disclosure generally relates to dental restorations and more particularly relates to a system and a method for estimating a crown pose using a neural network in restorative workflows.

### BACKGROUND OF THE INVENTION

Restorative dentistry is a branch of dentistry that focuses on diagnosing, managing and treating diseases of teeth and their supporting structures. The restorative dentistry primarily aims on restoring function, integrity, and morphology of missing and damaged tooth structure. This may further include procedures like fillings, crowns, bridges, implants and dentures. Such procedures help to improve aesthetics as well as health of the teeth of a patient, thereby ensuring they may chew, speak and smile properly.

Typically, the restorative dentistry focuses on repairing or replacing the missing or damaged tooth. While restoring the shape, size, strength and appearance of the missing or damaged tooth, a crown or a cap may be placed over the missing or damaged tooth. Placing the crown or the cap may facilitate crown restoration. Further, the crown restoration may require accurate determination of a location, an orientation, and a position for optimal placement of the crown in the oral cavity of a subject. Conventionally, the location of the crown may be determined using a virtual three-dimensional (3D) model of the teeth of the subject. In such a method, the 3D model may be segmented into teeth and gingiva, thereby facilitating to determine the crown position. For example, two neighbouring segments to a restorative site (for example, the missing or damaged tooth) in a jaw may be used to identify the crown location. Specifically, two natural teeth segments in a jaw around the missing or damaged tooth may be used to identify the crown location by interpolating the position of the restoration site. Using the neighbour segments to interpolate (and for second molars extrapolate) the estimated crown position may be both imprecise and restrictive due to inaccuracies of the interpolation process.

Typically to improve interpolation accuracies, a principal component analysis (PCA) model of estimated tooth poses of both jaws has been used. In such a case, providing partial information of the natural teeth may facilitate interpolation and extrapolation of the position of all remaining teeth. Further, the PCA model is more permissive though the precision drops off if the crown is to be placed far from natural teeth. Therefore, the PCA process includes a requirement that the restorative site has at least one natural tooth neighbour, to ensure that far placed natural teeth from the crown position will not appear. Moreover, the PCA model behaves poorly with any invalid input that would result in a useless crown. Such an invalid input may be obtained from previously applied algorithms that may for example be an output from a segmentation and a tooth identification algorithm. Further, if the segmentation is wrong, the PCA model may be prone to errors caused by a wrong segmentation of the teeth.

Therefore, there is a need for improved techniques for dental restoration to overcome the limitations associated with conventional methods for the dental restoration using segmentation and interpolation of the 3D model of dental structures of the oral cavities. Especially there is a need for improved techniques for determining the crown position in a restorative workflow.

### SUMMARY

A computer-aided dental restoration system, a method and a computer programmable product are provided. The computer-aided dental restoration system may allow estimation of a crown pose for dental restoration using neural network.

It is an objective of the present disclosure to provide techniques for computer-aided dental restoration system for estimating accurate crown pose and representing a virtual crown in three-dimensional (3D) model of a dentition of a patient, thereby overcoming the limitations associated with conventional methods for dental restoration.

In one aspect, a computer implemented method for estimating a crown pose and representing a virtual crown in a 3D model of a detention of patient is presented. The method comprises obtaining the 3D model of the dentition of the patient. The 3D model represents one or more teeth of the patient. The method comprising segmenting the 3D model to obtain segmented teeth and/or gingiva data. The method comprising generating an encoded 3D model representation suitable for processing by a trained neural network. The method for generating the encoded 3D model representation comprising subsampling a point cloud representation based on the segmented tooth data, the point cloud representation including a plurality of points representing the one or more teeth of the patient in the 3D model. The method further comprises determining, for each of the plurality of points, a surface normal representation. The method further comprises retrieving a dental notation of a restorative site. The method further comprises encoding each of the plurality of points with the corresponding surface normal representation and with a value relative to the restorative site to produce an encoded 3D model representation. The method further comprises inputting the encoded 3D model representation into the trained neural network. Further, the method comprises producing an output, using the trained neural network. The output comprises a prediction of a translation of each point of the encoded 3D model representation into positions corresponding to a crown pose. A processing step further comprises applying a registration optimization to determine the estimated crown pose as a local coordinate system and representing a retrieved virtual crown in the 3D model rendered in a graphical user interface utilizing the local coordinate system to automatically transform the retrieved virtual crown into the estimated crown pose in the 3D model.

The local coordinate system may define X, Y, Z directions and an origin for placing the virtual crown, for example at the restorative site.

Throughout the disclosure, term "encode" may be understood as "associate", "combine", "join" or "couple". Thus, to encode a point with a corresponding surface normal representatio may be understood as coupling or joining the point with the corresponding surface normal. On a practical level, if a point has coordinates (x, y, z) and the corresponding surface normal has components (nx, ny, nz), then the point encoded/joined with the surface normal may be (x, y, z, nx, ny, nz).

In an embodiment, the 3D model comprises a plurality of facets representing the one or more teeth of the patient. The subsampling further comprises generating a point cloud of the plurality of facets representing the one or more teeth.

In an embodiment, the surface normal representation may be determined as a surface normal of each of the plurality of facets representing the one or more teeth in the 3D model.

In an embodiment, the surface normal representation may be determined from calculating a point variance of each of the plurality of points of the subsampled point cloud.

In an embodiment, the retrieved virtual crown may be configured as a library virtual crown retrieved from a storage medium or an automated generated crown retrieved from a down-stream processing step.

In an embodiment, the method further comprises retrieving regular tooth site notations; and encoding each of the plurality of points associated with natural teeth at increasing distance from the restorative site dental notation with increasing integer values based on the regular tooth site notations. A positive integer value indicates a first direction from the restorative site and a negative integer value indicates a second direction opposite to the first direction from the restorative site. With this the method ensures that the restorative site is provided as the "zero tooth" while neighbouring teeth at increasing distance on both sides of the "zero tooth" of the restorative site is given the positive and negative increasing integer values allowing knowledge about a jaw direction when input to the trained neural network. With this, the estimation of the crown pose from of the neural network will have the "zero tooth" of the restorative site as the focus point, as the neural network, as will be apparent throughout has been trained to find the correct restorative site pose.

In an embodiment, the increasing integer values and decreasing integer values may be determined by subtracting the dental notation from the regular tooth site notation. In this way an automated generation of the direction of teeth in a jaw is achieved.

In an embodiment, the method further comprises encoding a first set of the plurality of points of the point cloud associated with a first part of the dentition with a first jaw value; and encoding a second set of points of the plurality of points of the point cloud associated with a second part of the dentition with a second jaw value. This allows the automated processes described herein to easily learn what is the upper jaw and lower jaw in an input dataset.

Accordingly, in an embodiment, the first jaw value is associated with an upper jaw of the dentition, and the second jaw value is associated with a lower jaw of the dentition.

In an embodiment, the method further comprises encoding each of the plurality of points of the point cloud associated with at least one of: one or more regular teeth, and/or a restorative site, with a distalness value.

In an embodiment, the method comprises encoding the distalness value, wherein the distalness value encoding including one or more first labelling points of teeth associated with a central incisor with a first label and one or more second labelling points associated with one or more distal teeth or one or more crown sites identifications from the central incisor with consecutive numbering.

In an embodiment, the method further comprises encoding a set of points from the plurality of points associated with a specified tooth with a plurality of different labels, wherein each of the plurality of different labels comprise at least one of: one or more of natural tooth, crown preparation, implant holes, scan body, healing abutment, post preparation, core preparation or other variations of preparation types.

In an embodiment, the local coordinate system comprises an origin, an X-direction pointing along a jaw arch of the dentition, a Y-direction pointing in an occlusal direction and a Z-direction pointing in a buccal direction.

The origin of the local coordinate system may be a consistent reference point associated with a corresponding crown, or a crown when placed at the restorative site. Such reference point may be a centroid, center of mass, surface center of mass, or a similar geometrically characteristic point.

In an embodiment, the Y-direction is common for each of the one or more teeth in the 3D representation.

In an embodiment, the trained neural network comprises a point-based neural network architecture.

In an embodiment, the estimated crown pose comprises a 4 x 4 affine rigid transformation matrix encoding for a global coordinate system to crown pose transformation applied to each of the plurality of points of an input point cloud. This ensures that each of the points of the input point cloud is aligned with the global coordinate system.

In an embodiment, the estimated crown pose comprises a coordinate system defining x, y, and z coordinates such that a virtual crown is configured to be positioned and oriented in the graphical user interface on the 3D model representation.

Generally, the term "pose" may be understood as a coordinate system with a specific origin and three mutually orthogonal exes intersecting in the origin.

In an embodiment, the method comprises utilizing a training phase configured to train the neural network. The training phase comprises obtaining a plurality of training 3D models of a plurality of dentitions, wherein each of the plurality of training 3D models is associated with teeth; segmenting the plurality of training 3D models to obtain segmented training tooth data; generating an encoded training dataset suitable for processing by the neural network during the training, wherein a method for generating the encoded training dataset comprises for each of the plurality of training 3D model, subsampling a point cloud representation based on the segmented tooth data, the point cloud representation comprising a plurality of points representing the one or more teeth of the patient in the training 3D models; determining, for each of the plurality of points, a surface normal representation; retrieving a dental notation of a restorative site and a local pose of the restorative site; encoding each of the plurality of points with the corresponding surface normal representation and with a value relative to the restorative site to produce an encoded 3D model representation. Such generated training dataset enables a neural network to learn in a training phase to find its way back to the local pose of the restorative site, as the retrieved local pose may be considered the "correct location" of the crown of for example a missing tooth. The local pose of the restorative site is an already known pose of a crown, as the training dataset comprises amongst other data 3D models with restorative sites, where a crown local pose has been identified.

The method for generating a training loop, may comprise applying a random transformation to a point cloud representation fed into the training loop, wherein the random transformation is configured to transforming the plurality of points of the point clouds into a new position with respect to the local pose of the restorative site; feeding the transformed point cloud into the neural network; and producing an output from the neural network. The output comprises a prediction of a translation of each point of the encoded training dataset representation into positions corresponding to a crown pose; comparing the produced output with the local pose of the restorative site; adjusting one or more neural network parameters based on the comparison; and repeating the training loop for the plurality of virtual 3D representations and applying a stopping criterion when the neural network meets a set threshold requirement for training. In other words, the training phase compares the estimated crown pose with the local pose and evaluates if the crown pose estimate matches closely the local pose.

In another aspect a computer-aided dental restoration system is provided. The system includes a scanner device configured to obtain 3D scan data of an intraoral site of a patient, and one or more processors, connected to the scanner device, configured to utilize the 3D scan data to generate a 3D model of the intraoral site of the patient. The one or more processors are configured to obtain the 3D model of the dentition of the patient, wherein the 3D model represents one or more teeth of the patient. The one or more processors are configured to segment the 3D model to obtain segmented tooth data. The one or more processors are configured to generate an encoded 3D model representation suitable for processing by a trained neural network. The method for generating the encoded 3D model representation includes subsampling a point cloud representation based on the segmented tooth data, the point cloud representation comprising a plurality of points representing the one or more teeth of the 3D model; determining, for each of the plurality of points, a surface normal representation; retrieving a dental notation of the restorative site; encoding each of the plurality of points with the corresponding surface normal representation and with a value relative to the restorative site and/ or the central incisors to produce an encoded 3D model representation; inputting the encoded 3D model representation into the trained neural network; producing an output, using the trained neural network, wherein the output comprises a prediction of a translation of each point of the encoded 3D model representation into positions corresponding to a crown pose. A processing step of the encoded 3D model representation further comprises applying a registration optimization to determine the estimated crown pose as a local coordinate system and representing the virtual crown in the 3D model rendered in a graphical user interface utilizing the local coordinate system to automatically transform a retrieved virtual crown into the estimated crown pose in the 3D model.

In yet another aspect, a computer programmable product is provided. The computer programmable product comprises a non-transitory computer readable medium having stored thereon computer executable instructions, which when executed by a processing circuitry, cause the processing circuitry to carry out operations, the operations comprises obtaining the 3D model of the dentition of the patient. The 3D model represents one or more teeth of the patient. The operation comprises segmenting the 3D model to obtain segmented tooth data. The operation comprises generating an encoded 3D model representation suitable for processing by a trained neural network. The operation for generating the encoded 3D model representation comprises subsampling a point cloud representation based on the segmented tooth data, the point cloud representation comprising a plurality of points representing the one or more teeth of the patient in the 3D model. The operation further comprises determining, for each of the plurality of points, a surface normal representation. The operation further comprises retrieving a dental notation of a restorative site. The operation further comprises encoding each of the plurality of points with the corresponding surface normal representation and with a value relative to the restorative site to produce an encoded 3D model representation. The operation further comprises inputting the encoded 3D model representation into the trained neural network. Further, the operation comprises producing an output, using the trained neural network. The output comprises a prediction of a translation of each point of the encoded 3D model representation into positions corresponding to a crown pose. A processing step of the encoded 3D model representation further comprises applying a registration optimization to determine the estimated crown pose as a local coordinate system and representing the virtual crown in the 3D model rendered in a graphical user interface utilizing the local coordinate system to automatically transform a retrieved virtual crown into the estimated crown pose in the 3D model.

The foregoing summary is illustrative only and is not intended to be in any way limiting. In addition to the illustrative aspects, embodiments, and features described above, further aspects, embodiments, and features will become apparent by reference to the drawings and the following detailed description.

As described, a computer-aided dental restoration system, a method and a computer programmable product are provided. One of the purposes of the present disclosure is to utilize a trained neural network to estimate a crown pose. This may address robustness requirements, lack of precision and versatility of existing principal component analysis (PCA) model. Further, the trained neural network performs with regular teeth close to crown position as well as with bad input obtained from other processing algorithm used to generate the dataset to be encoded. Such input may be an output from for example a segmentation processing and a tooth identification algorithm. Further, the disclosed method for dental restoration may facilitate precise, robust, and time efficient crown pose estimation using the trained neural network.

The disclosed system may leverage the use of trained neural network to automatically estimate the position of a crown in a 3D virtual model. The 3D virtual model may be generated from intraoral scan data obtained using e.g. an intra-oral scanner. Further, the disclosed method is faster and more efficient than fitting of the PCA model and interpolation process. Moreover, the disclosed method includes training an algorithm for cases with at least one preparation and cases without preparations. For example, the cases without preparations may indicate a missing tooth, and therefore the algorithm may perform well across any dataset where teeth are either missing in full or partly missing, such as for example when preparations are present in the 3D data of the patients' jaws.

The disclosed method may obtain upper and lower jaw models of an oral cavity of a patient. Thereafter, segmentation of the jaw models may be performed, thereby segmenting each of the jaws into teeth and gingiva. The method may employ an algorithm determining a jaw segmentation. Further, a tooth id (also denoted as the dental notation of the restorative site) of the crown may be determined to estimate the crown placement. Thereafter, determine whether the crown preparation exists or not using a toggle. The trained neural network may leverage the use of such inputs to determine a local coordinate system for the specified crown. The local coordinate system defines X, Y and Z directions for the crown and the coordinate system's origin. The X direction of the coordinate system points along the jaw, approximately in the direction of the tooth with the next lower number, according to a universal numbering system of teeth. The Y direction points in occlusal direction and is common for all tooth types, and the Z direction points in buccal direction. The local crown poses are defined as the tooth pose of the crown restoration, i.e., the axes follow the natural teeth/crowns. The global crown poses are defined with a common up/occlusal/Y direction for all teeth in a jaw and the X direction is defined as for the local crown pose. However, to support rendering the jaw (as input for the AI crown) it is defined along the arch of the jaw.

### BRIEF DESCRIPTION OF THE DRAWINGS

The present disclosure is illustrated by way of example and not by way of limitation in the figures of the accompanying drawings, in which the like reference numerals indicate like elements and in which:
FIG. 1A illustrates an exemplary network environment in which a computer-aided dental restoration system is implemented, in accordance with an example embodiment;
FIG. 1B illustrates an example schematic diagram of the computer-aided dental restoration system of FIG. 1A, in accordance with different example embodiments;
FIG. 2 illustrates a block diagram of the computer-aided dental restoration system, in accordance with an example embodiment;
FIG. 3A FIG. 3B, FIG. 3C and FIG. 3D illustrate sequence diagrams for estimating a crown pose, in accordance with various example embodiments;
FIG. 4A, FIG. 4B, and FIG. 4C illustrate schematic diagrams for point cloud and facet representations, in accordance with various example embodiments;
FIG 5A illustrates a schematic diagram of a processed output from the trained neural network, where local crown pose is estimated;
FIG 5B illustrates a schematic diagram of a 3D model having missing teeth and used as input to the method in accordance with various example embodiments;
FIG. 6Aand FIG. 6B illustrate exemplary schematic diagrams for retrieving regular tooth site notations, in accordance with different example embodiments;
FIG. 7A, FIG. 7B and FIG. 7C illustrate exemplary schematic diagrams for estimating the crown pose as a local coordinate system, in accordance with various example embodiments;
FIG. 8A, FIG. 8B, FIG. 8C, FIG. 8D, FIG. 8E, and FIG. 8F illustrate exemplary schematic diagrams for estimating the crown pose, in accordance with various example embodiments;
FIG. 9 illustrates a flowchart for training a neural network of the computer-aided dental restoration system, in accordance with an example embodiment;
FIG. 10 illustrates a flowchart of a computer-implemented method for estimating a crown pose, in accordance with an example embodiment;
FIG. 11 illustrates a flowchart of a method for generating an encoded 3D model representation, in accordance with an example embodiment; and
FIG. 12 illustrates a flowchart of processing steps for generating the encoded 3D model representation, in accordance with an example embodiment.

### DETAILED DESCRIPTION

In the following description, for purposes of explanation, numerous specific details are set forth in order to provide a thorough understanding of the present disclosure. It will be apparent, however, to one skilled in the art that the present disclosure may be practiced without these specific details. In other instances, systems and methods are shown in block diagram form only in order to avoid obscuring the present disclosure.

Reference in this specification to "one embodiment" or "an embodiment" means that a particular feature, structure, or characteristic described in connection with the embodiment is included in at least one embodiment of the present disclosure. The appearance of the phrase "in one embodiment" in various places in the specification are not necessarily all referring to the same embodiment, nor are separate or alternative embodiments mutually exclusive of other embodiments. Further, the terms "a" and "an" herein do not denote a limitation of quantity, but rather denote the presence of at least one of the referenced items. Moreover, various features are described which may be exhibited by some embodiments and not by others. Similarly, various requirements are described which may be requirements for some embodiments but not for other embodiments.

Embodiments of the present disclosure will now be described more fully hereinafter with reference to the accompanying drawings, in which some, but not all, embodiments of the disclosure are shown. Indeed, various embodiments of the disclosure may be embodied in many different forms and should not be construed as limited to the embodiments set forth herein; rather, these embodiments are provided so that this disclosure will satisfy applicable legal requirements. Like reference numerals refer to like elements throughout. As used herein, the terms "data," "content," "information," and similar terms may be used interchangeably to refer to data capable of being transmitted, received and/or stored in accordance with embodiments of the present disclosure. Further, the terms "processor", "controller" and "processing circuitry" and similar terms may be used interchangeably to refer to the processor capable of processing information in accordance with embodiments of the present disclosure. Further, the terms "electronic equipment", "electronic devices" and "devices" are used interchangeably to refer to electronic equipment monitored by the system in accordance with embodiments of the present disclosure. Thus, use of any such terms should not be taken to limit the spirit and scope of embodiments of the present disclosure.

The embodiments are described herein for illustrative purposes and are subject to many variations. It is understood that various omissions and substitutions of equivalents are contemplated as circumstances may suggest or render expedient but are intended to cover the application or implementation without departing from the spirit or the scope of the present disclosure. Further, it is to be understood that the phraseology and terminology employed herein are for the purpose of the description and should not be regarded as limiting. Any heading utilized within this description is for convenience only and has no legal or limiting effect.

As used in this specification and claims, the terms "for example" "for instance" and "such as", and the verbs "comprising," "having," "including" and their other verb forms, when used in conjunction with a listing of one or more components or other items, are each to be construed as open ended, meaning that that the listing is not to be considered as excluding other, additional components or items. Other terms are to be construed using their broadest reasonable meaning unless they are used in a context that requires a different interpretation.

A computer-aided dental restoration system, a method and a computer programmable product are provided for estimating accurate crown pose and representing a virtual crown in three-dimensional (3D) model of a dentition of a patient that is computationally inexpensive and power saving.

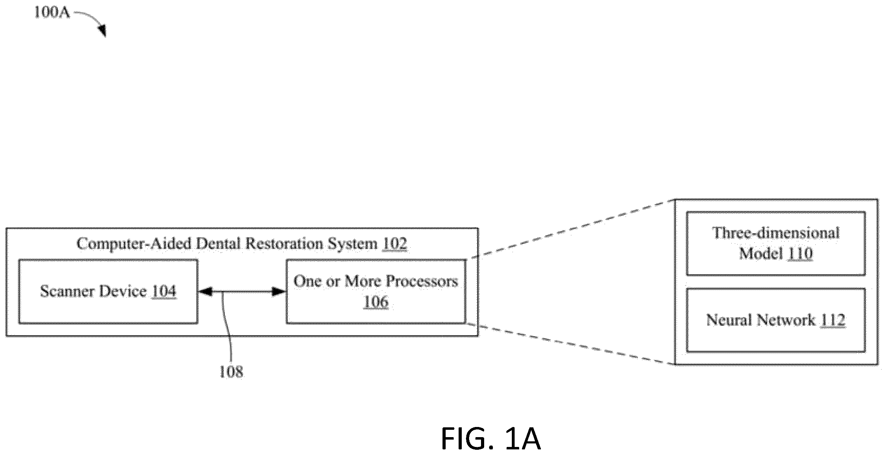

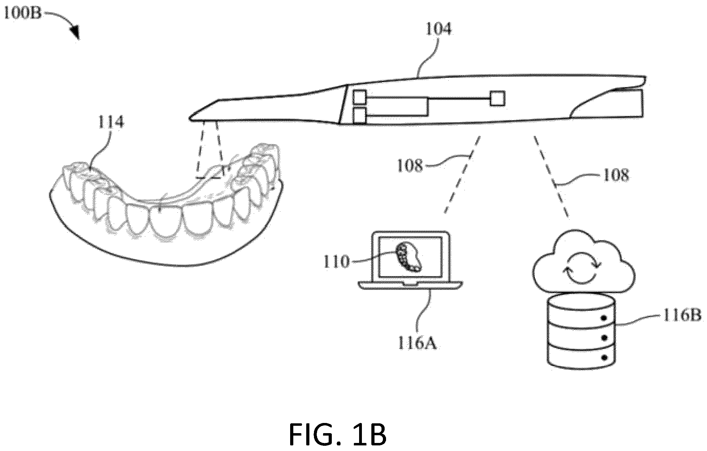

For instance, an exemplary network environment of the computer-aided dental restoration system for estimating crown pose is provided below with reference to FIG. 1A, and FIG. 1B.

FIG. 1A illustrates an exemplary network environment 100A in which a computer-aided dental restoration system 102 is implemented, in accordance with an example embodiment. The computer-aided dental restoration system 102 may be used to estimate crown pose(s) and represent a virtual 3D model of a dentition of a patient in a graphical user interface. Furthermore, the computer-aided dental restoration system may be used to position a virtual crown in the virtual 3D model in accordance with the estimated crown pose(s). For example, the computer-aided dental restoration system 102 may be used by a user, such as a person having knowledge of dentistry, for example, a dentist, a dental technician, and so forth. Further, it is possible that one or more components of the network environment 100A and/or the computer-aided dental restoration system 102 may be rearranged, changed, added, and/or removed without deviating from the scope of the present disclosure.

The computer-aided dental restoration system 102 comprises a scanner device 104, and one or more processors 106. The network environment 100A may further include communication channels 108 that may be configured to establish communicative coupling between components of the computer-aided dental restoration system 102, i.e., the scanner device 104 and the one or more processors 106 (referred to as the processors 106, hereinafter).

For example, the processors 106 may be embodied as one or more of various hardware processing means such as a coprocessor, a microprocessor, a controller, a digital signal processor (DSP), a processing element with or without an accompanying DSP, or various other processing circuitry including integrated circuits such as, for example, an ASIC (application specific integrated circuit), an FPGA (field programmable gate array), a microcontroller unit (MCU), a hardware accelerator, a special-purpose computer chip, or the like. As such, in some embodiments, the processors 106 may include one or more processing cores configured to perform independently. A multi-core processor may enable multiprocessing within a single physical package. Additionally, or alternatively, the processors 106 may include one or more processors configured in tandem via a bus to enable independent execution of instructions, pipelining and/or multithreading. Additionally, or alternatively, the processors 106 may include one or more processors capable of processing large volumes of workloads and operations to provide support for big data analysis. In an example embodiment, the processors 106 may be in communication with the other components of the computer-aided dental restoration system 102 (referred to as system 102, hereinafter) via a bus or the communication channel 108 for passing information among components of the system 102.

In an example, when the processors 106 are embodied as an executor of software instructions, the instructions may specifically configure the processors 106 to perform the algorithms and/or operations described herein when the instructions are executed. However, in some cases, the processors 106 may be a processor specific device (for example, a mobile terminal or a fixed computing device) configured to employ an embodiment of the present disclosure by further configuration of the processors 106 by instructions for performing the algorithms and/or operations described herein. The processors 106 may include, among other things, a clock, an arithmetic logic unit (ALU) and logic gates configured to support operation of the processors 106. The network environment, such as, 100A may be accessed using the communication channel 108.

The communication channel 108 may be wired, wireless, or any combination of wired and wireless communication networks, such as cellular, wireless fidelity (Wi-Fi), internet, local area networks, or the like. In accordance with an embodiment, the communication channel 108 may be one or more wireless full-duplex communication channels. In one embodiment, the communication channel 108 may include one or more networks such as a data network, a wireless network, a telephony network, or any combination thereof. It is contemplated that the data network may be any local area network (LAN), metropolitan area network (MAN), wide area network (WAN), a public data network (e.g., the Internet), short range wireless network, or any other suitable packet-switched network, such as a commercially owned, proprietary packet-switched network, e.g., a proprietary cable or fibre-optic network, and the like, or any combination thereof. In addition, the wireless network may be, for example, a cellular network and may employ various technologies including enhanced data rates for global evolution (EDGE), general packet radio service (GPRS), global system for mobile communications (GSM), Internet protocol multimedia subsystem (IMS), universal mobile telecommunications system (UMTS), etc., as well as any other suitable wireless medium, e.g., worldwide interoperability for microwave access (WiMAX), Long Term Evolution (LTE) networks (for e.g. LTE-Advanced Pro), 5G New Radio networks, ITU-IMT 2020 networks, code division multiple access (CDMA), wideband code division multiple access (WCDMA), wireless fidelity (Wi-Fi), wireless LAN (WLAN), Bluetooth, Internet Protocol (IP) data casting, satellite, mobile ad-hoc network (MANET), and the like, or any combination thereof. The scanner device 104 may be configured to communicate with the processors 106 via the communication channel 108.

The system 102 may be configured to store, such as in a memory, data generated by the system 102. For example, the system 102 may be configured to store data captured by the scanner device 104, i.e., 3D scan data of an intraoral site of the patient. In an example, the 3D scan data may be sensed, measured or determined by the scanner device 104. The 3D scan data further comprises signal information associated with the intraoral site of the patient. Moreover, the system 102 may be configured to store data generated by the processors 106. Such data may comprise a three-dimensional (3D) representation, i.e., a three-dimensional (3D) model 110 of the intraoral site of the patient.

The system 102 may be utilized for registration of intraoral scans and generating the 3D model of the dentition of the patient to show surface information and/or inner layers of the dental object. The system 102 may include multiple components, such as the scanner device 104, the processors 106 and memory (not shown) that may communicate with each other to obtain the intraoral scans of the dental object.

In an example, the scanner device 104 may include the processors 106. In another example, the scanner device 104 may be coupled to the processors 106, wherein the processors 106 may be located remotely and may perform operations associated with the system 102. The system 102 may have enhanced processing capabilities that may be required to process the 3D scan data in real time to generate the 3D model 110 of the dentition of the patient.

The system 102 may have enhanced processing capabilities that may be required to process the plurality of two-dimensional (2D) images in real time to generate plurality of 2D internal feature images and further overlay the 2D internal features image information (or 2D internal features) of the dentition of the patient onto the 3D surface model.

The scanner device 104 may be configured to capture the plurality of 2D images during a scanning session of the dentition of the patient. The plurality of 2D images may indicate images of a dental object of the patient captured using light that is emitted at wavelength corresponding to a range of IR or near-infrared (NIR) wavelengths. The plurality of 2D images may include images of the dental object of the subject from various viewing angles or viewing points. Further, the plurality of 2D images may include the visible light images indicating images of the dental object of the subject captured using light that is emitted at wavelength corresponding to a range of white light wavelengths or a selected sub spectrum of the visible light such as blue light (400 - 495 nm), green light (490 - 570 nm), and fluorescence light (300 - 800nm). The plurality of 2D images may also include images of the dental object of the subject from various viewing angles. In certain cases, the plurality of 2D images may also include images captured at other visible and/or non-visible light wavelengths.

In operation, the processors 106 are configured to receive and/or obtain signal information from one or more sensors (referred to as sensors, hereinafter) of the scanner device 104. The sensors of the scanner device 104 may include, for example, light sensors and/or image sensors. To this end, the based-on light sensed by the sensors, the sensors may capture and provide the signal information corresponding to the dentition of the patient to the processors 106. Thereafter, the processors 106 are configured to obtain the 3D scan data of the intraoral site of the patient based on the signal information.

In an example, the scanner device 104 may include a web server that may be configured to communicate via a web network and establish a connection to the communication channel 108. The scanner device 104 may be configured to execute the web server to provide the 3D scan data obtained from the sensors to the processors 106. The scanner device 104 may further include a processing unit, a memory unit, a communication interface, the sensors, and additional components. The processing unit, the memory unit, the communication interface, and the additional components may be communicatively coupled to each other. Details of the components of the scanner device 104 are further provided, for example, in FIG. 1B.

The system 102 may further include a neural network 112. The neural network 112 may be a computational network or a system of artificial neurons, arranged in a plurality of layers, as nodes. The plurality of layers of the neural network 112 may include an input layer, one or more hidden layers, and an output layer. Each layer of the plurality of layers may include one or more nodes (or artificial neurons). Outputs of all nodes in the input layer may be coupled to at least one node of the hidden layer(s). Similarly, inputs of each hidden layer may be coupled to outputs of at least one node in other layers of the neural network. Outputs of each hidden layer may be coupled to inputs of at least one node in other layers of the neural network. Node(s) in the final layer may receive inputs from at least one hidden layer to output a result.

The number of layers and the number of nodes in each layer may be determined from hyper-parameters of the neural network. Such hyper-parameters may be set before or while training the neural network 112 on a training dataset. Each node of the neural network 112 may correspond to a mathematical function (e.g., a sigmoid function or a rectified linear unit) with a set of parameters of the neural network 112. The set of parameters may include, for example, a weight parameter, a regularization parameter, and the like. Each node may use the mathematical function to compute an output based on one or more inputs from nodes in other layer(s) (e.g., previous layer(s)) of the neural network. All or some of the nodes of the neural network 112 may correspond to the same or a different mathematical function.

In the training of the neural network 112, one or more parameters of each node of the neural network 112 may be updated based on whether an output of the final layer for a given input (from a training dataset) matches a correct result (i.e later described as the retrieved local pose of the restorative site) based on a loss function for the neural network 112. The above process may be repeated for the same or a different input until a minimum loss function may be achieved, and a training error may be minimized. Several methods for training are known in the art, for example, gradient descent, stochastic gradient descent, batch gradient descent, gradient boost, meta-heuristics, and the like.

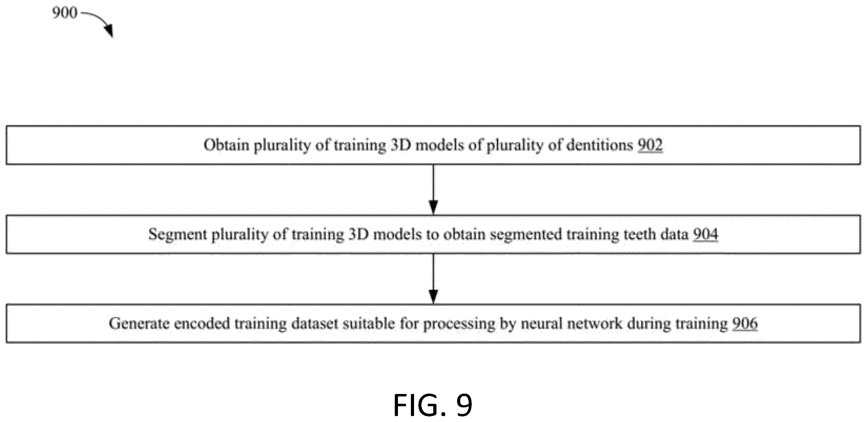

The neural network 112 may include electronic data, such as, for example, a software program, code of the software program, libraries, applications, scripts, or other logic or instructions for execution by a processing device, such as circuitry. The neural network 112 may be implemented using hardware including a processor, a microprocessor (e.g., to perform or control the performance of one or more operations), a field-programmable gate array (FPGA), or an application-specific integrated circuit (ASIC). Alternatively, in some embodiments, the neural network may be implemented using a combination of hardware and software. Although in FIG. 1, the neural network 112 is shown integrated within the processor 106, the disclosure is not so limited. Accordingly, in some embodiments, the neural network 112 may be a separate entity in the processor 106, without deviation from the scope of the disclosure. Examples of the neural network 112 may include, but are not limited to, an untrained neural network (UNN), an artificial neural network (ANN), a deep neural network (DNN), a convolutional neural network (CNN), a fully connected neural network, and/or a combination of such networks. Details about the neural network 112 are provided, for example, in FIG. 9.

FIG. 1B shows an example schematic diagram 100B of the system 102, in accordance with an example embodiment. The system 102 includes the scanner device 104 configured to obtain the 3D scan data. In an example, the scanner device 104 may emit light of various wavelengths. For example, the images of the dentition 114 may be captured using light having different wavelengths, such as white light, blue light, IR light, NIR light, fluorescent light, etc. To this end, visible light images and/or the IR images for the dental object may be captured from a same position and at same time due to high pulse repetition rate of the light emitted from the scanner device 104.

The 3D scan data generated by the scanner device 104 may be communicated to the processors 106 over wired or wireless communication channel 108. In an example, the processors 106 may be implemented as a computing device 116A or a server 116B external to the scanner device 104.

The system 102 includes the one or more processors 106 arranged in the scanner device 104, the external computer 116A and/or the server 116B. The scanner device 104 may include a processor, and the processor is configured to process sensor data from sensors of the scanner device 104 into information, such as the 3D scan data configured to be transmitted to the external computer 116A and/or the server 116B. Furthermore, the external computer 116A and/or the server 116B may include the processors 106 to process the received signal information to obtain the 3D scan data.
For example, the subject may require a dental treatment. In such a case, the system 102 may be utilized by a user, such as a dentist to provide the dental treatment to the subject. In an embodiment, the subject may be present at a dental clinic. In such a case, the system 102 may be utilized in a treatment room of the dental clinic. In another embodiment, the subject may have been requested for a home visit for the dental treatment. In such a case, the system 102 may be utilized in the home of the subject. To start the dental treatment, the scanner device 104 may be utilized by the user to obtain the 3D scan data of the intraoral site, using the light sensor.

In an example, throughout a scanning of the dentition of the patient, the projector unit may be configured to constantly emit the light signal (for example, the infrared light signal or the visible light signal). The scanner device 104 may include a projector unit configured to emit light at different wavelengths, such as at near-infrared wavelength, infrared wavelength, white-coloured wavelengths and/or coloured visible wavelengths onto the dentition 114. In an example, the projector unit may be configured to emit light with different wavelengths in a pulsating manner during different time periods onto the dentition 114, wherein the different wavelengths include a near-infrared wavelength, an infrared wavelength, and a visible wavelength.

In an example, the visible light emitted by the projector unit may include one or more colour lights of the filtered visible light signals, such as red, green, blue, and white. The projector unit may include multiple light sources that are configured to emit the one or more colours lights and the infrared light. The multiple light sources may be arranged within a single module that includes multiple Light Emitting Diodes (LEDs) that are configured to emit different wavelengths within the visible and non-visible wavelength ranges. In another example, light source, i.e., one or more LEDs that may be configured to emit infrared light, may be arranged separately from the light source that is configured to emit the visible light.

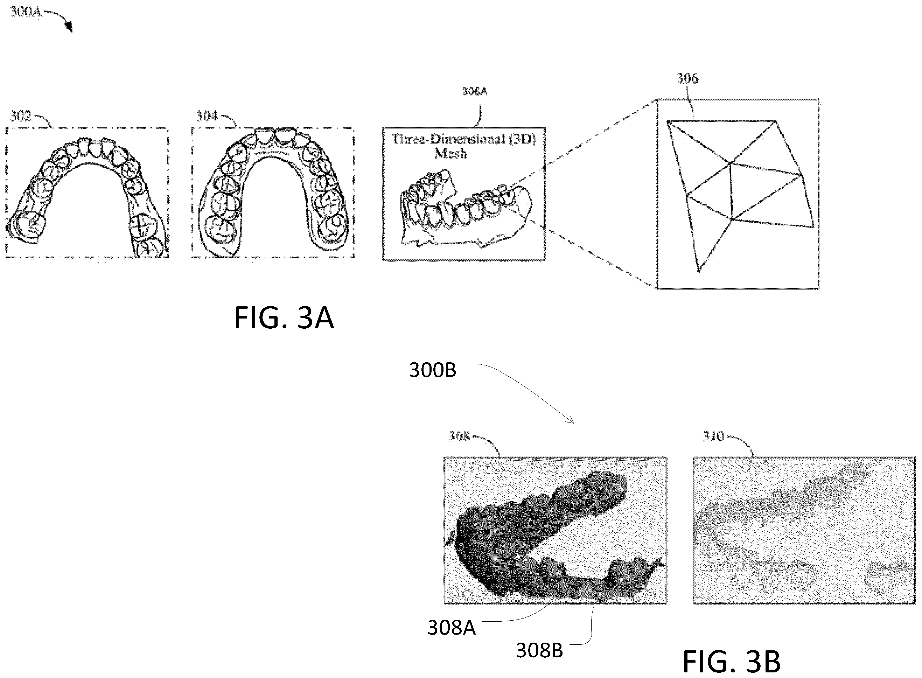

The scanner device 104 may be configured to obtain the 3D scan data of the intraoral site of the patient. Further, the processor 106, connected to the scanner device 104, may be configured to utilize the 3D scan data to generate the 3D model 110 of the dentition of the patient. In an example, the 3D model 110 represents one or more teeth and/or gingiva of the patient. Upon obtaining the 3D model 110 of the dentition of the patient, the processor 106 may segment the 3D model 110 to obtain segmented tooth data, wherein the segmented tooth data comprises a segmentation of the scan data into individual teeth, and gingiva. Thereafter, the processor 106 may facilitate generating an encoded 3D model representation based on the segmented tooth data, which encoded 3D model is configured to besuitable for processing by the trained neural network 112. Details associated with a method for generating the encoded 3D model representation are provided, for example, in FIG. 3A.

The encoded 3D model representation is provided as an input to the trained neural network 112 to produce an output comprising a prediction of a translation of each point of the encoded 3D model 110 representation into positions corresponding to a crown pose. Further, the process comprises applying a registration optimization to determine the estimated crown pose as a local coordinate system. Thereafter, a virtual crown representation of the 3D model is retrieved and rendered in a graphical user interface utilizing the local coordinate system to automatically transform the retrieved virtual crown into the estimated crown pose in the 3D model.

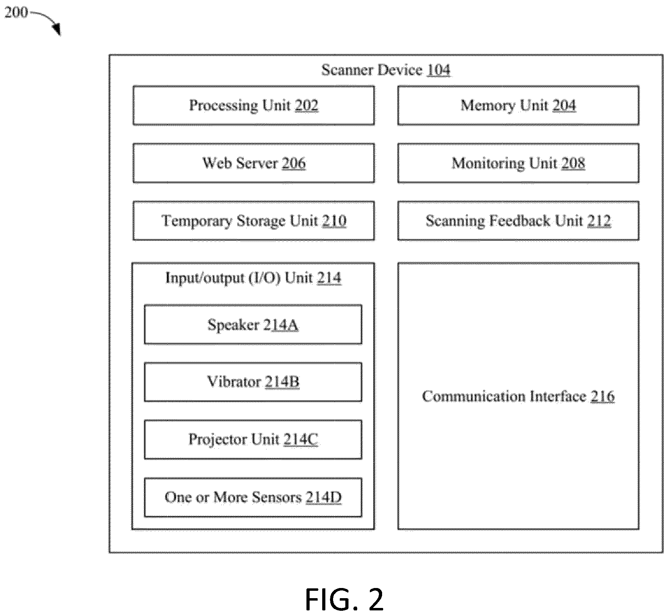

FIG. 2 illustrates a block diagram 200 of the scanner device 104, in accordance with an example embodiment. FIG. 2 is explained in conjunction with elements of FIG. 1A, and FIG. 1B. The scanner device 104 may include at least one processing unit (hereinafter, also referred to as "processing unit 202"), a memory unit 204, a web server 206, a monitoring unit 208, a temporary storage unit 210, a scanning feedback unit 212, an input/output (I/O) unit 214, and a communication interface 216.

The processing unit 202 may be embodied in a number of different ways. For example, the processing unit 202 may be embodied as one or more of various hardware processing means such as a coprocessor, a microprocessor, a controller, a digital signal processor (DSP), a processing element with or without an accompanying DSP, or various other processing circuitry including integrated circuits such as, for example, an ASIC (application specific integrated circuit), an FPGA (field programmable gate array), a microcontroller unit (MCU), a hardware accelerator, a special-purpose computer chip, or the like. In an embodiment, the processing unit 202 may be embodied as a high-performance microprocessor having series of System on Chip (SOCs) which includes relative powerful and power-efficient Graphics Processing Units (GPUs) and Central Processing Units (CPUs) and a small form factor. As such, in some embodiments, the processing unit 202 may include one or more processing cores configured to perform independently. A multi-core processor may enable multiprocessing within a single physical package. Additionally, or alternatively, the processing unit 202 may include one or more processors configured in tandem via the bus to enable independent execution of instructions, pipelining and/or multithreading.

In some embodiments, the processing unit 202 may be configured to detect light signal (for example, NIR and/or visible light), using the I/O unit 214 during the scanning session of teeth of a subject, such as a patient requiring a dental treatment. The detected NIR and visible light may be used to generate the plurality of images of the dentition 114 or jaw of the patient from various angles or viewing points. In an example embodiment, the processing unit 202 may be in communication with the memory unit 204 via a bus for passing information among components of the scanner device 104.

The memory unit 204 may be non-transitory and may include, for example, one or more volatile and/or non-volatile memories. In other words, for example, the memory unit 204 may be an electronic storage device (for example, a computer readable storage medium) comprising gates configured to store data (for example, bits) that may be retrievable by a machine (for example, a computing device like the processing unit 202). The memory unit 204 may be configured to store information, data, content, applications, instructions, or the like, for enabling the apparatus to carry out various functions in accordance with an example embodiment of the present disclosure. For example, the memory unit 204 may be configured to store the detected light signal after the scanning session of the teeth is finished. The detected light signal after the scanning session may be stored as 3D scan data. In certain cases, the memory unit 204 may be configured to store the compressed 3D scan data. As exemplarily illustrated in FIG. 2, the memory unit 204 may be configured to store instructions for execution by the processing unit 202. As such, whether configured by hardware or software methods, or by a combination thereof, the processing unit 202 may represent an entity (for example, physically embodied in circuitry) capable of performing operations according to an embodiment of the present disclosure while configured accordingly. Thus, for example, when the processing unit 202 is embodied as the microprocessor, the processing unit 202 may be specifically configured hardware for conducting the operations described herein. Alternatively, as another example, when the processing unit 202 is embodied as an executor of software instructions, the instructions may specifically configure the processing unit 202 to perform the algorithms and/or operations described herein when the instructions are executed. The processing unit 202 may include, among other things, a clock, an arithmetic logic unit (ALU) and logic gates configured to support operation of the processing unit 202. It should be noted that the processing unit described in relation to the scanning device may preferably process the scan data to ensure generation of the 3D data that are used for rendering of a 3D model in a graphical user interface of a computing device (116A of Figure 2) separate to the scanning the device. The method of estimating a crown pose described herein may mainly be run on a processor of the computing device and/or a cloud server operatively connected to the computing device.

The web server 206 may be a software, a hardware, or a combination thereof that may be configured to store and provide data to a web browser associated with the processors 106. For example, the 3D scan data be provided to the web browser of the processors 106 via the web server 206. As the web server 206 may be accessed by any web browser, the need for installation of an additional software by the processors 106, to connect to the web server 206 may be eliminated. The web server 206 may communicate to one of the communication channels 108 via a web network. In an example, the web server 206 and the processors 106 may communicate to a common wireless full-duplex communication channel via the web network for transmission and reception of the visible light information and the IR information. The web server 206 and the web browser may communicate via, for example, Hypertext Transfer Protocol (HTTP), Simple Mail Transfer Protocol (SMTP), or File Transfer Protocol (FTP). Once the web server 206 and the web browser are connected, the web server 206 may provide a web application on the web browser.

The monitoring unit 208 may be a software, a hardware, or a combination thereof that may be configured to monitor a bandwidth of one of the communication channels 108 (such as the wireless full-duplex communication channel) via which the scanner 104 and the processors 106 may be connected. Moreover, the monitoring unit 208 may be configured to monitor a connection of one of the communication channels 108 via which the scanner device 104 and the processors 106 may be connected.

The temporary storage unit 210 may be a software, a hardware, or a combination thereof that may be configured to store the 3D scan data and colour information when the bandwidth of the communication channels 108 (such as the wireless full-duplex communication channel) is determined to be below the minimum bandwidth. The temporary storage unit 210 may further transmit the stored 3D scan data to the processors 106 when the bandwidth is determined to be above or equal the minimum bandwidth. Examples of the temporary storage unit 210 may include, but may not be limited to, a random-access memory (RAM), or a cache memory.

The scanning feedback unit 212 may be a software, a hardware, or a combination thereof that may be configured to receive status input from the monitoring unit 208. Based on the received status input, the scanning feedback unit 212 may provide a scanning feedback signal to the user, such as a dentist, of the scanner device 104. In an embodiment, the scanning feedback signal is used to provide guidance to the user regarding an area of the teeth where a scanning quality of the scanning session is low and adequate visible light information and/or the NIR information of the is not received. For example, the scanning feedback unit 212 may provide the scanning feedback signal as, for example, an acoustic feedback signal, a haptic feedback, or a visual feedback.

The I/O unit 214 may include circuitry and/or software that may be configured to provide output to the user of the scanner device 104 and receive, measure or sense input information. The I/O unit 214 may include a speaker 214A, a vibrator 214B, a projector unit 214C, and one or more sensors 214D. In an embodiment, the speaker 214A may be configured to output the acoustic feedback signal to guide the user. The vibrator 214B may be, for example, a transducer configured to convert the scanning feedback signal that may be an electrical signal into a mechanical output, such as the haptic feedback in form of vibrations to guide the user.

As may be understood, the scanner device 104 may be configured to detect the IR and the visible light that may be reflected from the teeth of the subject. In this regard, the projector unit 214C may be configured to output one or more visible or white coloured wavelength pulses, and one or more IR wavelength pulses. For example, the visible wavelength pulses and the IR wavelength pulses may be cast onto the dentition 114 or the teeth to illuminate the teeth of the subject, such as a patient. Further, the visible light wavelength pulses and the IR wavelength pulses may be reflected or refracted from the surface and/or the inner region of the teeth. The one or more sensors 214D may be configured to detect visible coloured wavelength pulses and IR wavelength pulses that may be reflected and/or refracted from the surface or inner region of the teeth. In an example, the one or more sensors 214D may include one or more light sensors, such as event sensor or image sensor. For example, the image sensors may correspond to a camera that may be configured to generate the 3D mode 110 based on the illumination of the teeth using the IR and the visible light.

The communication interface 216 may comprise input interface and output interface for supporting communications to and from the scanner device 104. The communication interface 216 may be a device or circuitry embodied in either hardware or a combination of hardware and software that is configured to receive and/or transmit data to/from the scanner 104. In this regard, the communication interface 216 may include, for example, an antenna (or multiple antennae) and supporting hardware and/or software for enabling communications with a wireless communication network. Additionally, or alternatively, the communication interface 216 may include the circuitry for interacting with the antenna(s) to cause transmission of signals via the antenna(s) or to handle receipt of signals received via the antenna(s). In some environments, the communication interface 216 may alternatively or additionally support wired communication. As such, for example, the communication interface 216 may include a communication modem and/or other hardware and/or software for supporting communication via cable, digital subscriber line (DSL), universal serial bus (USB) or other mechanisms.

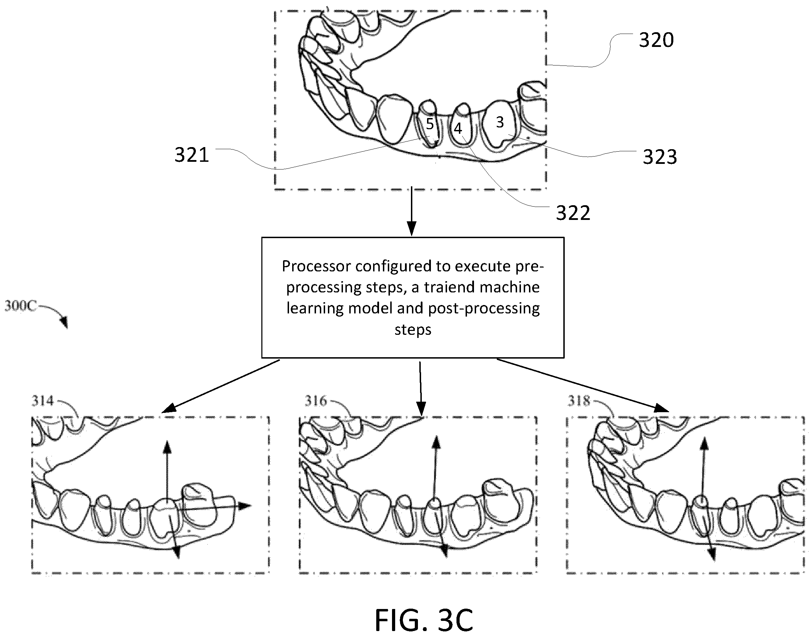

FIG. 3A, FIG. 3B, and FIG. 3C illustrate sequence diagrams 300A, 300B and 300C, respectively, for estimating a crown pose, in accordance with an example embodiment. FIG. 3A, FIG. 3B, and FIG. 3C are explained in conjunction with elements of FIG. 1A, FIG. 1B, and FIG. 2 in the following.

In an embodiment, the processor 106 is configured to obtain the 3D model of the dentition of the patient. In an example, the projector unit 214C may illuminate the dental object of the oral cavity using visible light, white light wavelength pulses and/or IR and/or NIR wavelength pulses. In an example, the dentition 114 (or dental object) may correspond to one or more structures, such as tooth, a set of teeth, and/or gums inside the oral cavity of the subject. Upon illuminating the oral cavity, the one or more sensors of the scanner device 104 may detect visible light, and/or IR and/or NIR. Such detected visible light and/or NIR may be reflected or refracted from surface or inner region of the dentition 114. In other words, the one or more sensors may detect the signal information reflected or refracted from the dentition 114 or tooth. For example, the one or more sensors may include image sensors that may be configured to capture the visible light images based on the detected visible light information and the IR images based on the detected IR information. In this manner, the one or more sensors may be configured to generate the plurality of 2D images of the dentition 114. Such plurality of 2D images may be processed to determine the 3D surface information for the dentition 114. In an example, in-focus measurements of the plurality of 2D images may be determined. Further, projected features of the dentition 114 may be tracked across the plurality of 2D images. For example, a correspondence function may be performed or solved to triangulate depth information for the dentition 114 and determine the 3D surface information for the dentition 114.

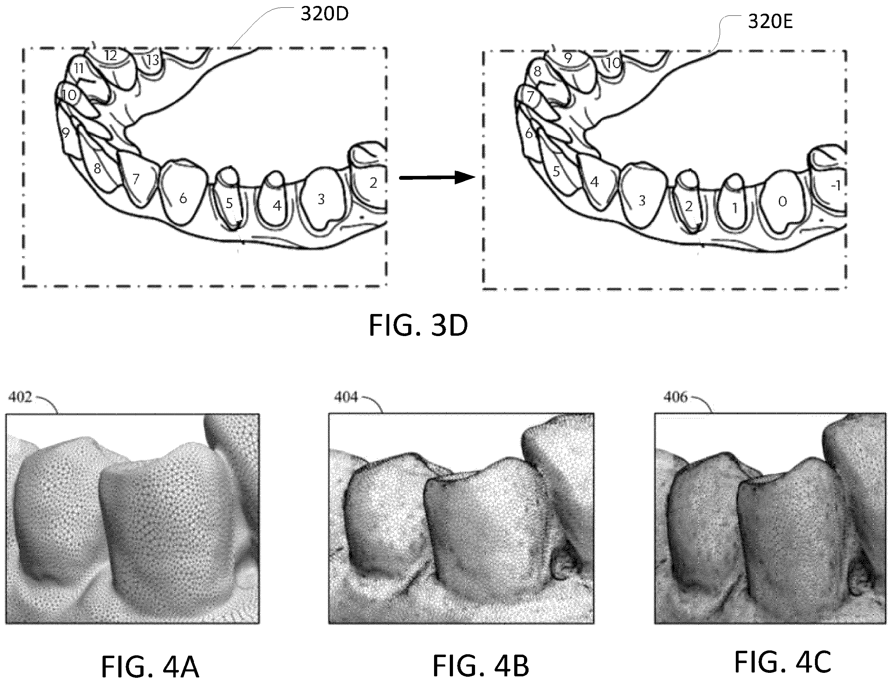

Thereafter, the 3D model is obtained for the dentition of the patient based on the 3D surface information. Examples of the obtained 3D is illustrated in Figure 3, where 302 illustrates a first jaw of the dentition and the 304 illustrates a second jaw of the dentition. In an example, a 3D patch may be generated corresponding to a part of a surface of the dentition 114, or a surface of the set of teeth of the subject by accessing calibration data of the one or more sensors and transforming the 3D surface information corresponding to the part into real-world 3D coordinates and texture information. For example, different 3D patches may be generated for different parts of the surface of the teeth using the plurality of 2D images. In certain cases, there may be an overlap in parts covered by different 3D patches. For example, the 3D patch for the part of the surface of the teeth may be registered or associated with one or more previously generated 3D patches for other parts and/or overlapping parts of the surface of the teeth by locating corresponding data points. Thereafter, the 3D patch for the part may be fused with the one or more previously generated 3D patches relating to other parts of the surface of the teeth to generate the 3D model. Further, the 3D model represents one or more teeth of the patient. As explained, in an example, illustrated in Figure 3A the 3D model may include an upper jaw 302 and a lower jaw 304 as acquired from the signal information from the intraoral scanner (scanner device 104) and transformed into a 3D model.

In an embodiment, the 3D model 306A provided as an example comprises a plurality of facets 306 representing the one or more teeth of the patient as illustrated in Figure 3A. In an example, each tooth of the 3D models illustrated in Figure 3A represents a segment configured with the plurality of facets 306. Further, for each facet of the plurality of facets 306 of a segment of the 3D model, a facet normal (also denoted as a surface normal) is determined. Accordingly, along with the coordinates of the sampled points of a 3D point cloud, the facet normal at the sample locations as determined are stored. The facets of the 3D representation may be represented by a point in the 3D point cloud. Further, the 3D point cloud may include 3D points or 3D control points within voxels in a signed distance field, and the signed distance field may be converted into a 3D mesh 306A for rendering the 3D model. In an example, the 3D model is generated as an offset mesh 306A comprising a grid or mesh 306A of 3D control points. For example, an initial grid or mesh 306A of 3D control points for generating the 3D model may be created over input images, i.e., the plurality of 2D images. The grid may be a grid of points or a set of key points (referred to as 3D control points) that are strategically placed based on a type of distortion or transformation to be performed on the images. These 3D control points may be used as references for mapping pixels of images in the corresponding mesh 306A to generate the 3D model. For example, each of the 3D control points may provide associated offset information that defines how much the pixel at that control point should be moved or adjusted. The 3D model may also include colour data and texture data of the surface of the teeth.

In an example, the facet refers to flat, polygon surface element that makes up mesh 306A of 3D model. These facets 306 may be quadrilateral and are components of the mesh 306A that approximates the surface of scanned object. When the dentition 114 is scanned using the scanner device 104, a scan data is typically processed to create a mesh, such as the mesh illustrative represented in Figure 3A, where the 3D model 306A comprises a mesh of interconnected plurality of facets 306. Such a mesh model 306A represents surface geometry of the dentition 114 to create interconnected facets. In an example, a quality and resolution of the scan data may affect a number and a size of the plurality of facets 306 in the mesh model 306A. Generally, a large number of the plurality of facets indicates a more detailed and accurate representation of the surface of the dentition 114.

In an example, determining the plurality of facets 306 in the scan data involves data acquisition processing and mesh model 306A generation. The scanner device 104 may be used to capture the geometry of the dentition 114. Thereafter the scanner device 104 collects point cloud data, which is a set of points on the dental objects surface. Once the scan data is acquired, point cloud processing is done. Noise and outliers from the point cloud data may be removed using noise reduction. Further, the scan data includes multiple scan data taken from different angles. Such multiple scan data may be aligned to create a single unified point cloud. Secondly, the point cloud may be converted into polygonal mesh 306A using algorithms like Delaunay triangulation, Poisson surface and ball pivoting algorithm. In an example, the Delaunay triangulation constructs a mesh 306A of the tetrahedra from the point cloud. The Poisson surface may generate a smooth, watertight mesh 306A and ball pivoting algorithm may roll a virtual ball over the point cloud to connect points into triangle.

In an example, the 3D model could be considered as a 3D point cloud where no facets would be present. In an example, the 3D model of upper jaw 302 and lower jaw 304 is loaded into a computer. Further, the 3D models have been generated from scan data acquired by an intra-oral scanner as previously described.

Referring to FIG. 3B, there is shown an example jaw 308, where two teeth 308A, 308B are missing. The model could also have been with a preparation, meaning that part of the teeth is still present.

In an embodiment, the 3D model may be segmented to obtain segmented tooth data. Accordingly, the 3D model may be segmented, and 3D point cloud 310 may be generated as illustrated in Figure 3B. For the 3D model loaded into the system 102 a 3D point cloud is generated and/or a segmentation step is provided before or after generation of the point cloud to separate teeth from gingiva. The gingiva information may potentially not be used in the solution. For the example jaw 308, an example of a point cloud 310 without gingiva may be constructed.. The point cloud 310 without gingiva may be produced by segmentation enabling a separation of gingiva from teeth and identifying teeth in the 3D model. In an example, the scan data from the intraoral scanner (scanner device 104) is segmented into teeth and gingiva, where the gingiva parts of the input jaws are removed from both jaws.

Referring to FIG 3C, an output of processing steps described herein is illustrated. To produce the output illustrated in Figure 3C, an encoded 3D model representation of the 3D model has been processed by a trained neural network and subsequent processing steps described herein and in the following. The encoded 3D model representation may be generated as a substantial post-processing step and input to the trained neural network 112. The 3D point cloud generated from a post-processing step may be encoded with a plurality of different information for each point which is used as input for the trained neural network, as described throughout.

As previously described, the system 102 is configured to retrieve restorative site identification, which is preferable to allow the neural network to know which teeth are missing, contains preparation or needs to be restored in some fashion. In Figure 3C the 3D model 320 is given as an example. The 3D model 320 is configured to be input to a processor which comprises executable means for performing one or more pre-processing steps, inputting the post-processed 3D model to the trained neural network and subsequently post-process the out to estimate the crown pose of missing teeth, preparations etc. in the 3D model 320.In the example given in Figure 3C, the 3D model 320 comprises three preparations 321, 322, 323, where at least parts of the teeth are missing, and for which the method described herein is able to estimate correct crown poses for. Each of the preparation 321, 322, 323 is considered to have a restorative site identification in the form of a dental notation number, which for these cases in one example could be in accordance with the universal number system meaning that the dental notations of the restorative sites would be for 5 for preparation 321, 4 for preparation 322 and 3 for preparation 323. The restorative site identification is used to encode the points associated with the restorative site with a value (e.g., zero) indicating that a tooth is missing or damaged (resulting in a preparation) for these points. The dental notations 3, 4, 5 for the restorative sites 321, 322, 323 are used to encode the 3D model with zero points for the restorative site being looked at and for which the neural network estimates a crown pose. Accordingly, if the method described herein is to process the dental model 320 to estimate the crown pose for the restorative site having dental notation 3, then the encoded 3D model would comprise a zero label for points associated with the dental notation 3, allowing the neural network to know that this is the teeth that are being looked at. The encoded 3D model with the restorative site identified is input to the neural network.

Thereafter, the output from the trained machine learning model is on a high level an estimated crown pose, where the pose defines an orientation (i.e. coordinate system) of where the crown should be placed, as will be explained.

The method for generating the encoded 3D model representation comprises subsampling a point cloud representation based on the segmented tooth data, the point cloud representation comprising a plurality of points representing the one or more teeth of the 3D model.

As previously mentioned, in case the input 3D model 320 comprises more than one preparation site or missing teeth, the machine learning model will be run for each preparation site 321, 322, 323, where the preparation site for each machine learning model run is provided as the teeth to be looked at, for example, if the jaw 320 is as show in FIG. 3C, where the dental notations are illustrated as 3, 4, 5 for the restorative preparation sites 321, 322, 323 all comprises a preparation, then the machine learning model will be run 3 times with first dental notation 3 being the restorative site, second the dental notation 4 being the restorative site, and a third time with the dental notation 5 being the restorative site. Running the jaw 320 through the method described herein will produce the resulting crown poses illustrated as a coordinate system in 3D models 314, 316 , 318 as illustrated in Figure 3C. For example, 3D model 314 illustrates an output when the dental notation 3 of 3D model 320 is the restorative site, 3D model 316 illustrates an output when dental notation 4 of 3D model 320 is the restorative site, and 3D model318 illustrate an output when dental notation 5 of 3D model 320 is the restorative site. In particular, 314, 316 and 318 illustrate the output from the machine learning model for each run of the jaw 320 and as can be seen the machine learning model and subsequent processing is able to return the estimated crown pose for all dental notations 3, 4 and 5 very precisely in their corresponding iteration.

The subsampling further comprises generating a point cloud of the plurality of facets representing the one or more teeth. Then determining, for each of the plurality of points, a surface normal representation. In an embodiment, the surface normal representation is determined as a surface normal of each of the plurality of facets representing the one or more teeth in the 3D model.

Further, the surface normal representation is determined from calculating a point variance of each of the plurality of points of the subsampled point cloud. In an embodiment, encoding each of the plurality of points of the point cloud associated with at least one of: one or more regular teeth, or a restorative site, with a distalness value.

The crown pose estimation from the machine learning model and the post-processing described herein may be used to arrange a library crown in the 3D model. Alternatively, it may be used to position an automated generated crown in the 3D model using the estimated crown pose. Furthermore, other usages could be considered to include implant work. Where a crown pose is to be estimated to evaluate a drilling direction or similar.

As the suggested solution utilizes a trained neural network, a training dataset is generated to train the network on. The data used to train the neural network on, as previously described comprises cases with at least one preparation and cases without preparations. Accordingly, the 3D models used in the training of the neural network comprises data where a crown has already been positioned on the 3D model, and therefore the optimal crown location, i.e. zero coordinate for the crown location is known. This known zero coordinate is also describes the local pose retrieved during training of the neural network.

Using the training data, where the crown location (zero coordinate) is known (also denoted as the local pose for training purposes), the training data set is generated. The 3D models used in the training phase and (including upper and lower jaw) as acquired from the scan data from an intra-oral scanner is segmented into teeth and gingiva, where the gingiva parts of the input jaws may be removed from both jaws. The 3D models, when generated from the scanner comprises facets, where each tooth represents a segment configured with a plurality of facets, as previously explained. The 3D model could be considered as a point cloud where no facets would be present.

Subsequently, the remaining segments (i.e. teeth) are sampled into a common point cloud or alternatively separate point clouds of each tooth. In other words, a step comprises subsampling a point cloud representation of the segmented teeth into a plurality of points representing one or more teeth of the 3D model.

In case of the 3D model representation comprising facets, then for each facet of a segment of the 3D model, a facet normal (also denoted as a surface normal) is determined. Accordingly, along with the coordinates of the sampled points of the point cloud, the facet normals at the sample locations as determined are stored.

In an alternative, where the 3D model is a point cloud, the surface normals may also be found by determining the point variances (using e.g. a principal component analysis (PCA) model) of the point cloud. Determining the surface normals and training the network on surface normal information allows the network to learn directions associated with teeth in the 3D model.

Furthermore, the sampled points of the point cloud may be encoded with the additional information. For, example encoding with the difference of dental notations from the segment difference to that of the crown tooth, resulting in a zero value at the preparation (which could be a restorative site) and increasing integer values at increasing distance to the crown and with the sign reflecting the side of the crown.

If using the situation illustrated in Figure 3C for training purposes, then coordinates systems of dental notation 3, 4 and 5 would be considered the known local poses used in the training phase to generate the encoded 3D model. This part of the method ensures that the dental notation of the restorative site (for example teeth with dental notation 3 in Figure 3C) is used to label the 3D model with a zero point for the restorative site. This zero point is obtained by subtracting the dental notation of the restorative site from the dental notation itself to receive a zero label for the restorative site. In the example given in Figure 3C, the dental notation of the restorative site is for example dental notation 3 and the zero point is found by subtracting dental notation 3 as the restorative site from the dental notation 3 achieving the zero point of the restorative site to be associated with dental notation 3. Furthermore, the dental notation of the restorative site is used to subtract the dental notation no. from the remaining teeth in increasing distance of the restorative site on both sides of the restorative site. In this way the teeth of the jaw associated with the restorative site will be provided with a zero number at the restorative site and increasing positive integer values at one site of the restorative site and increasing negative integer values at another site of the restorative site. This is in more detail illustrated in Figure 3D where 3D model 320D is provided with dental notations 2 to 13 for the teeth illustrated in the example 3D model. An example of the encoding of the 3D model in accordance with the described zero point of the restorative site being looked at is given in Figure 3D to the right. Accordingly, Figure 3D illustrates the process of retrieving the restorative site of the 3D model 320D, wherein the restorative site in the example given is associated with dental notation 3. In the processing of the data to generate the encoded 3D model 320E it is clear from the illustrative example that the tooth corresponding to dental notation 3 and being associated with the restorative site is provided with a zero value, wherein teeth to the right of the zero value has negative integer value and the teeth to the left of the zero value is provided with increasing positive integer values in accordance with a subtraction procedure of the restorative site dental notation from the dental notations of the remaining teeth in the 3D model.. This allows the network to learn a direction of the jaw.

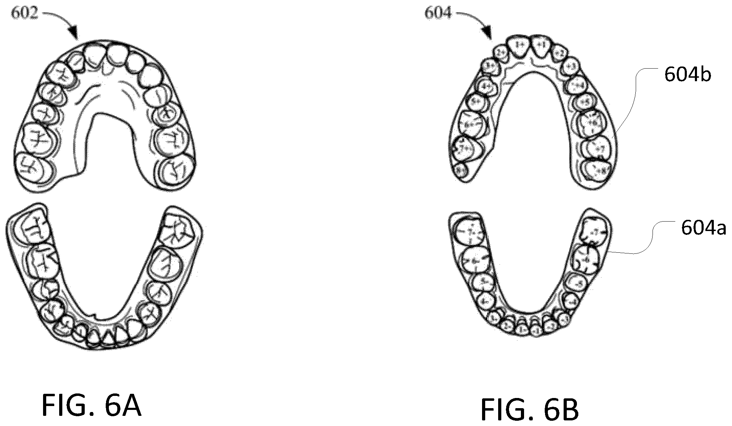

In addition to the previous described encoding of the 3D model, the 3D model may also in an example be encoded with jaw kind information. That is, the jaw kind is encoded as 0 or 1 for the upper or lower jaw, but it could be opposite way. Mesial-distal position encoded from 0 to 7 corresponding to central incisor to third molar, which may be one-hot encoded. The jaw encoding may comprise the same negative and positive increasing integer values as described above, as this allows the network to know which antagonist teeth are associated with each other and as illustrated in Figure 6B.

The segment type may in an example be divided into 7 categories, where e.g. crown prep, abutment and post and core preps are grouped. These types of segment types may be one hot encoded. After encoding the sampled points of the segmented 3D model facets into a point cloud, the point cloud with the encoded additional information is provided as input for a PointNet model that predicts the location of each point. The point net can be any suitable point based architecture, where examples include, but not limited to, PointNet, PointNet++, Assanet etc.

The prediction of each point is possible due to a processing step including generating a training loop, wherein for each point cloud of the training dataset, a random transformation of the point cloud is applied. This ensures that each time a point cloud is presented to the machine learning model during training, the points of the input point cloud is randomly transformed in a direction away from the known crown location into a new position with respect to the local coordinate of the crown location. This process is iterated for each input data in a randomized manner. This ensures that the points of the input data are moved away from the ground truth location of the crown (that is the known local pose) and when provided as input to the machine learning model during training, the machine learning model learns to estimate the optimal position of the crown location. In other words, the machine learning model is being trained to find its way back to the original location of the crown as given by the input training dataset. This means that every time a new transformation of the point cloud is applied to the same input data, this input is considered as a new input training data. So more than one training input data can be obtained from only one input by applying a new transformation every time. This way of training creates a bigger number of training data inputs. Accordingly, the machine learning model is configured to output predicted locations of all the points in an input point cloud. The predicted point locations correspond to applying the world to crown pose transformation to the points of the input point cloud.

As the predicted point locations do not agree to one rigid transformation, a further post-processing step is applied to find the optimal transformation for all points, using a registration optimization, which has been found to improve the algorithm performance significantly.

This optimization step may also be referred to as a transformation optimization, or even just a rotation optimization as only rotation is performed, utilized for the purpose of accurately determining the estimated crown pose as the local coordinate system. An example of the transformation optimization algorithm that may be used in this post processing step is the Kabsch algorithm.

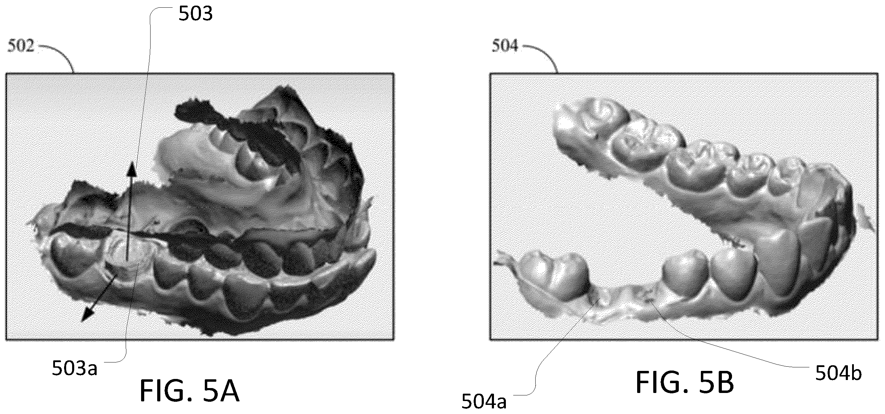

An input jaw (upper and lower jaw) transformed to the crown pose using the output from the method can be seen in an example in FIG. 5A, along with the points sampled from the meshes consisting of facets. The PointNet model may be used with a few modifications, primarily removing a softmax on the final output from the segmentation version is used. Other PointNet models could similarly be used.

Further, the distalness value encoding includes one or more first labelling points of teeth associated with a central incisor with a first label. Additionally, the distalness value encoding includes one or more second labelling points associated with one or more distal teeth from the central incisor with consecutive numbering

Further, encoding a first set of the plurality of points of the point cloud associated with a first part of the dentition with a first jaw value. Further, encoding a second set of points of the plurality of points of the point cloud associated with a second part of the dentition with a second jaw value. In an embodiment, the first jaw value is associated with an upper jaw of the dentition, and the second jaw value is associated with a lower jaw of the dentition

In an embodiment, the estimated crown pose comprises a 4 x 4 affine rigid transformation matrix encoding for a global coordinate system to crown pose transformation applied to each of the plurality of points of an input point cloud

FIG. 4A, FIG. 4B, and FIG. 4C illustrate schematic diagrams for point cloud and facet representations, in accordance with various example embodiment. FIG. 4A, FIG. 4B, and FIG. 4C are explained in conjunction with elements of FIG. 1A, FIG. 1B, FIG. 2, FIG. 3A, FIG. 3B and FIG. 3C.

FIG. 4A depicts an input 3D model 402 with facet representation. FIG. 4B depicts pre-processing the input 3D model 402 to generate a point cloud 404.

FIG. 4C depicts a representation 406 comprising point cloud 404 and facets 3D model 402 together. In an example, the 3D model 402 with facet representation may be overlayed on the generated point cloud 404 to produce the representation 406.

FIG. 5A illustrate a schematic diagram of a processed output from the trained neural network, where a local crown pose is estimated and FIG. 5B illustrates a schematic diagram of a 3D model having missing teeth and used as input to the method in accordance with various example embodiments.. FIG. 5A and FIG. 5B are explained in conjunction with elements of FIG. 1A, FIG. 1B, FIG. 2, FIG. 3A, FIG. 3B, FIG. 4A, FIG. 4B, and FIG. 4C.

FIG. 5A depicts an output with the estimated crown pose 503 of the method described herein. The scan data represented as the 3D model in Figure 5A may have been acquired using an intra-oral scanner, where the crown pose is estimated using the method described herein to generate the coordinate crown pose 503 of tooth 503a..

FIG. 5B depicts another example of a 3D model for which two crown poses of teeth 504a, 504b may be estimated using the method described herein. In an example, the processor 106 may be configured to segment the 3D model into tooth and gingiva, thereby obtaining the segmented tooth data that may be used to generate the encoded 3D model data used as input to the trained machine learning model. As should be understood by now, the method described herein ensures that for the example provided in Figure 5B two crown poses will be estimated to ensure that a subsequently retrieved virtual crown is automatically and correctly positioned in the 3D model of Figure 5B.

FIG. 6A and FIG. 6B illustrate exemplary schematic diagrams for retrieving regular tooth site notations, in accordance with different example embodiments. FIG. 6A and FIG. 6B are explained in conjunction with elements of FIG. 1A, FIG. 1B, FIG. 2, FIG. 3A, FIG. 3B, FIG. 4A, FIG. 4B, FIG. 4C, FIG.5A, and FIG. 5B.

FIG. 6A depicts an example of a full jaw 602 of a patient. FIG. 6B depicts tooth site notations 604 associated with teeth in the jaw. As seen especially in Figure 6B, each tooth may be provided with a dental notation (i.e. also denoted regular tooths site notation), where the central incisors are provided with the number 1 and neighbouring teeth at a distance to each central incisor is provided with an increasing integer value. Furthermore, as seen in Figure 6B, the upper jaw 604b is provided with positive integer values and the lower jaw 604a is provided with negative integer values. By utilizing the method described herein it is possible to encode the encoded 3D model with this jaw kind information (negative or positive values) and the tooth number to allow the model to recognize type of jaw (upper or lower) and/or the tooth direction from central incisors to last molar teeth of the jaw.

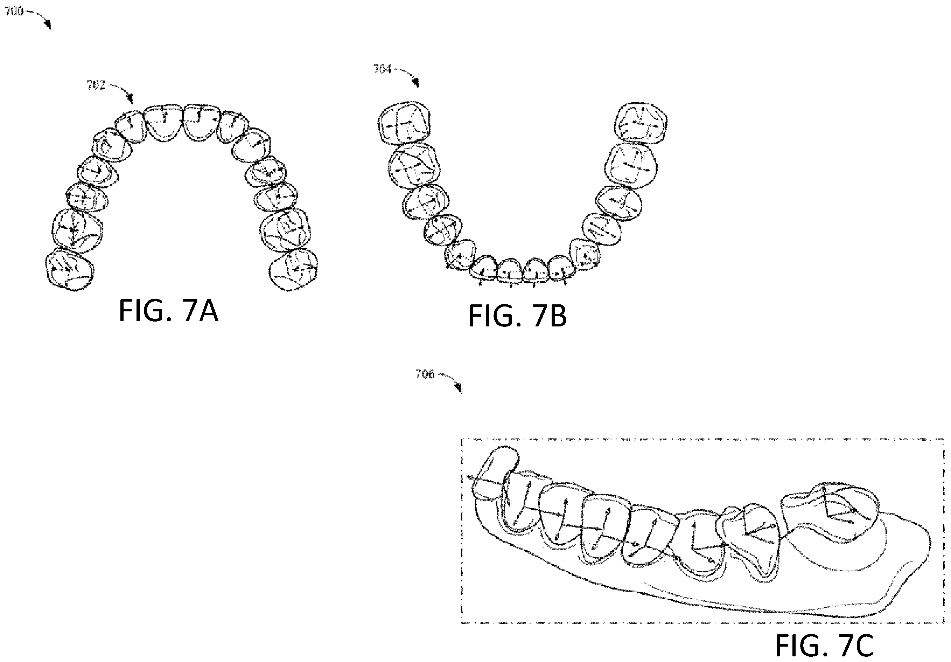

FIG. 7A, FIG. 7B and FIG. 7C illustrate exemplary schematic diagrams for estimating the crown pose as local coordinate system, in accordance with various example embodiments. FIG. 7A, FIG. 7B, and FIG. 7C are explained in conjunction with elements of FIG. 1A, FIG. 1B, FIG. 2, FIG. 3A, FIG. 3B, FIG. 4A, FIG. 4B, FIG. 4C, FIG.5A, FIG. 5B, FIG.6A, and FIG.6B.

FIG. 7A, depicts a coordinate system 702 for upper jaw of the patient. FIG. 7B depicts the coordinate system 704 for lower jaw of the patient. The coordinate systems 702 and 704 may be utilized to determine the crown pose

FIG. 7C depicts a local coordinate system 706 for estimated crown pose. The local coordinate system 706 includes an origin, i.e., an X-direction pointing along a jaw arch of the dentition, a Y-direction pointing in an occlusal direction and a Z-direction pointing in a buccal direction.

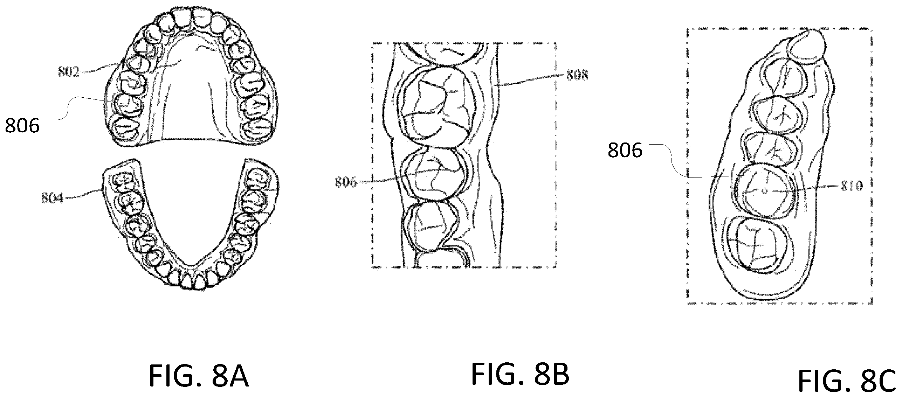

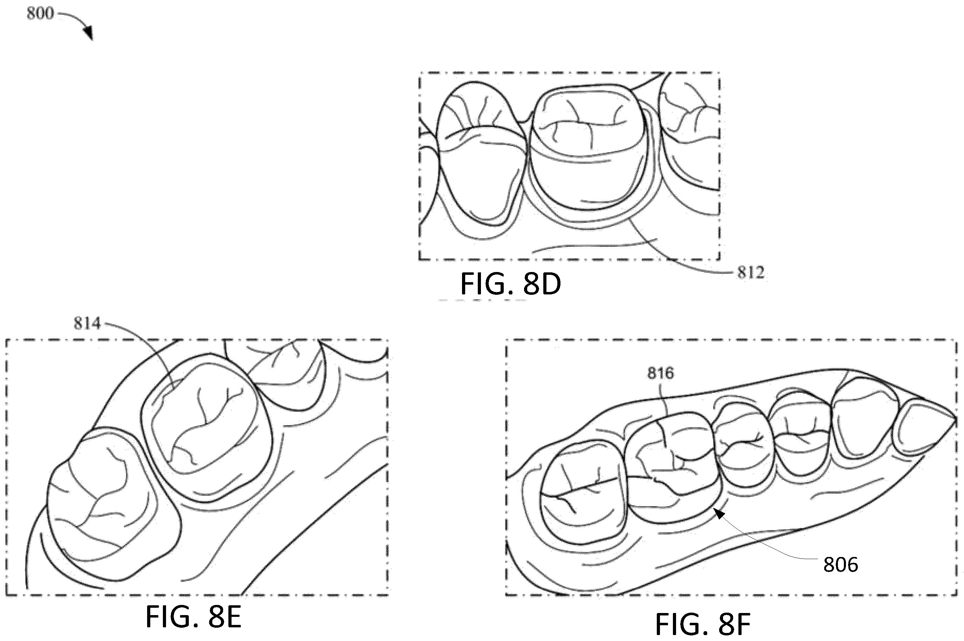

FIG. 8A, FIG. 8B, FIG. 8C, FIG. 8D, FIG. 8E, and FIG. 8F illustrate exemplary schematic diagram for estimating the crown pose, in accordance with various example embodiments. FIG. 8A, FIG. 8B, FIG. 8C, FIG. 8D, FIG. 8E, and FIG. 8F are explained in conjunction with elements of FIG. 1A, FIG. 1B, FIG. 2, FIG. 3A, FIG. 3B, FIG. 4A, FIG. 4B, FIG. 4C, FIG. 5A, FIG. 5B, FIG.6A, FIG.6B, FIG.7A, FIG.7B and FIG.7C.

Figures 8A to depicts example pre-processing steps for a restorative workflow, wherein the method described herein may be used.

FIG. 8A depicts an upper jaw 802 and a lower jaw 804 of a patient that has been scanned during a clinal visit, where a restorative workflow may be considered for at least one tooth. In an embodiment, the scanner device 104 may obtain the 3D scan data of the dentition of the patient. Thereafter, the processor 106 may obtain a 3D model representation of the dentition, which may include one or more teeth of the patient. For example, at least one tooth, exemplified by tooth 806 in Figure 8A may be selected for restoration. Thereafter, a type of restoration, for example, crown restoration, and the type of material of the crown restoration may be determined based on the restoration site.

In a restorative workflow several steps of working with the 3D model may be applied before reaching to the point where the crown estimation as described herein is performed. Some of these steps are illustrated in the following figures, where FIG. 8B depicts a scanning step 808 of the area of the 3D model 802, where the tooth 806 to be restored is identified. In a subsequent step, the restorative workflow generates a marking 810 of the tooth 806 on which restoration may be planned.

Subsequently, a separate module or program of crown modelling may be executed which is generally illustrated in FIG. 8D. In this non-limiting to the invention step, the tooth to be repaired with a restorative workflow is worked with in the 3D model to set a margin line 812. This separate module, which is independent of the method suggested herein is dedicated to setting the margin lines for the restoration site.

Subsequently, in a restorative type use case, an insertion direction 814 may be automatically generated as depicted in FIG. 8E. In a subsequent step, the restorative workflow may apply the method described herein to estimate the crown pose to which a retrieved virtual crown should be positioned. The outcome of this process is schematically illustrated in FIG. 8F.

FIG. 8F, where it is illustrate that a virtual crown 816 retrieved from e.g. a library set of crown have been fit onto the tooth 806, which were to be restored. In other words, in the example depicted in Figure 8F, a virtual crown 816 may be rendered in a graphical user interface at the position of the estimated crown pose. In an example, the system 102 may automatically position an example crown chosen based on the 3D scan data. Further, the type and material of the crown may be modified using an available option in a library associated with the machine learning software. In an embodiment, the retrieved virtual crown is configured as a library virtual crown retrieved from a storage medium or an automated generated crown retrieved from a down-stream processing step.

FIG. 9 illustrates a flowchart 900 for training the computer-aided dental restoration system, in accordance with an example embodiment. FIG. 9 is explained in conjunction with elements of FIG. 1A, FIG. 1B, FIG. 2, FIG. 3A, FIG. 3B, FIG. 4A, FIG. 4B, FIG. 4C, FIG. 5A, FIG. 5B, FIG.6A, FIG.6B, FIG.7A, FIG.7B, FIG.7C, FIG.8A, FIG.8B, FIG.8C, FIG.8D, FIG. 8D, FIG.8E and FIG.8F. The operations of the exemplary method may be executed by any computing system, for example, by the processors 106 of the system 102 of FIG. 1A. The operations of the flowchart 900 may start at 902.

At 902, a plurality of training 3D models of a plurality of dentitions may be obtained. Each of the plurality of training 3D models may be associated with teeth. In an embodiment, the trained neural network 112 includes a point-based neural network architecture. The method includes utilizing a training phase configured to train the neural network 112. Further, training phase include obtaining a plurality of training 3D models of a plurality of dentitions. Each of the plurality of training 3D models is associated with teeth.

At 904, the plurality of training 3D models may be segmented to obtain segmented training tooth data. The training phase include segmenting the plurality of training 3D models to obtain segmented training tooth data.

At 906, an encoded training dataset suitable for processing by the neural network during the training may be generated. Further, the training phase include generating an encoded training dataset suitable for processing by the neural network during the training. The method for generating the encoded training dataset includes for each of the plurality of training 3D model, subsampling a point cloud representation based on the segmented tooth data. The point cloud representation comprising a plurality of points representing the one or more teeth of the patient in the training 3D models. Further generating the encoded training dataset includes determining, for each of the plurality of points, a surface normal representation. Generating the encoded training dataset further includes retrieving a dental notation of a restorative. Each of the plurality of points with the corresponding surface normal representation and with a value relative to the restorative site may be encoded to produce an encoded 3D model representation. Further a method for generating the encoded training dataset includes subsampling, for each of the plurality of training 3D model, a point cloud representation based on the segmented tooth data, the point cloud representation including a plurality of points representing the one or more teeth of the patient in the training 3D models. Further, the method includes determining, for each of the plurality of points, a surface normal representation, and retrieving a dental notation of a restorative.

Thereafter, the method includes applying a random transformation to a point cloud representation fed into the training loop. The random transformation is configured to transforming the plurality of points of the point clouds into a new position with respect to the local pose of the restorative site. Further, the transformed point cloud may be fed into the neural network; and an output from the neural network may be produced. The output includes a prediction of a translation of each point of the encoded training dataset representation into positions corresponding to a crown pose. Thereafter, the produced output may be compared with the local pose of the restorative site, and one or more neural network parameters may be adjusted based on the comparison. In an example, the training loop for the plurality of virtual 3D representations and applying a stopping criterion when the neural network meets a set threshold requirement for training may be repeated to train the neural network.

Further, a method for generating a training loop includes applying a random transformation to a point cloud representation fed into the training loop. The random transformation is configured to transform the plurality of points of the point clouds into a new position with respect to the local pose of the restorative site. The method further includes feeding the transformed point cloud into the neural network; and producing an output from the neural network. The output includes a prediction of a translation of each point of the encoded training dataset representation into positions corresponding to a crown pose. Further, the output includes comparing the produced output with the local pose of the restorative site. The output includes adjusting one or more neural network parameters based on the comparison; and repeating the training loop for the plurality of virtual 3D representations and applying a stopping criterion when the neural network meets a set threshold requirement for training

Accordingly, blocks of the flowchart 900 support combinations of means for performing the specified functions and combinations of operations for performing the specified functions. It will also be understood that one or more blocks of the flowchart 900 can be implemented by special purpose hardware-based computer systems which perform the specified functions, or combinations of special purpose hardware and computer instructions.

Alternatively, the scanner device 104 may include means for performing each of the operations described above. In this regard, according to an example embodiment, examples of means for performing operations may include, for example, a processor and/or a device or circuit for executing instructions, such as the operations or instructions for controlling heat transfer in the scanner device 104.

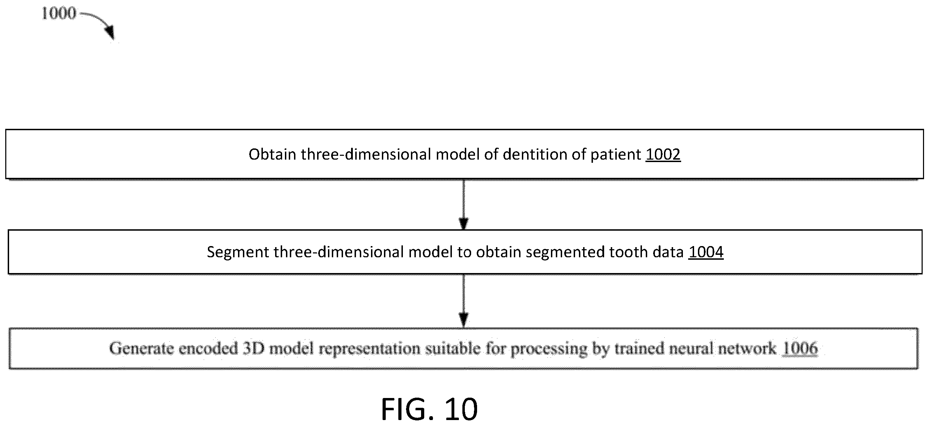

FIG. 10 illustrates a flowchart 1000 of a computer-implemented method for estimating a crown pose, in accordance with an example embodiment. FIG. 10 is explained in conjunction with elements of FIG. 1A, FIG. 1B, FIG. 2, FIG. 3A, FIG. 3B, FIG. 4A, FIG. 4B, FIG. 4C, FIG. 5A, FIG. 5B, FIG.6A, FIG.6B, FIG.7A, FIG.7B, FIG.7C, FIG.8A, FIG.8B, FIG.8C, FIG.8D, FIG. 8D, FIG.8E, FIG.8F, and FIG. 9. The operations of the exemplary method may be executed by any computing system, for example, by the processors 106 of the system 102 of FIG. 1A. The operations of the flowchart 1000 may start at 1002.

At 1002, the 3D model of the dentition of the patient may be obtained. The 3D model may represent one or more teeth of the patient. Further, the 3D model includes a plurality of facets representing the one or more teeth of the patient.

At 1004, the 3D model may be segmented to obtain segmented tooth data.

At 1006, an encoded 3D model representation suitable for processing by a trained neural network may be generated.

Accordingly, blocks of the flowchart 1000 support combinations of means for performing the specified functions and combinations of operations for performing the specified functions. It will also be understood that one or more blocks of the flowchart 1000 can be implemented by special purpose hardware-based computer systems which perform the specified functions, or combinations of special purpose hardware and computer instructions.

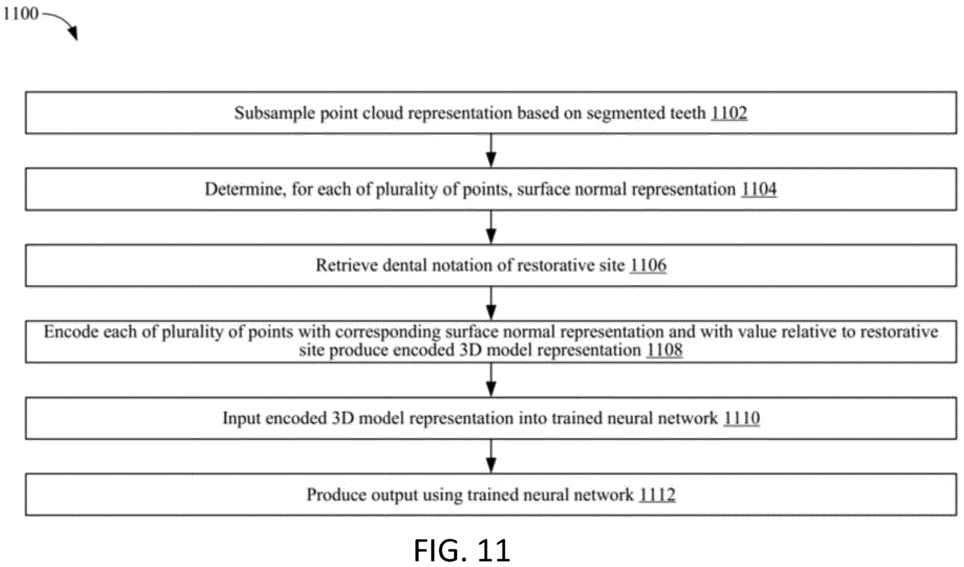

FIG. 11 illustrates a flowchart of a method for generating the encoded 3D model representation, in accordance with an example embodiment. FIG. 11 is explained in conjunction with elements of FIG. 1A, FIG. 1B, FIG. 2, FIG. 3A, FIG. 3B, FIG. 4A, FIG. 4B, FIG. 4C, FIG. 5A, FIG. 5B, FIG.6A, FIG.6B, FIG.7A, FIG.7B, FIG.7C, FIG.8A, FIG.8B, FIG.8C, FIG.8D, FIG. 8D, FIG.8E, FIG.8F, FIG. 9, and FIG. 10. The operations of the exemplary method may be executed by any computing system, for example, by the processors 106 of the system 102 of FIG. 1A. The operations of the flowchart 1100 may start at 1102.

At 1102, a point cloud representation may be subsampled based on the segmented tooth data. The point cloud representation may include a plurality of points representing the one or more teeth of the patient in the 3D model. Further, the subsampling includes generating a point cloud of the plurality of facets representing the one or more teeth.

At 1104, a surface model representation may be determined for each of the plurality of points. The surface normal representation may be determined as a surface normal of each of the plurality of facets representing the one or more teeth in the 3D model. Further, the surface normal representation may be determined from calculating a point variance of each of the plurality of points of the subsampled point cloud

At 1106, a dental notation of a restorative site may be retrieved. Further the retrieved virtual crown may be configured as a library virtual crown retrieved from a storage medium or an automated generated crown retrieved from a down-stream processing step

At 1108, each of the plurality of points with the corresponding surface normal representation and with a value relative to the restorative site may be encoded to produce an encoded 3D model representation.

At 1110, the encoded 3D model representation may be inputted into the trained neural network. Further, the trained neural network comprises a point-based neural network architecture.

At 1112, an output, using the trained neural network, may be produced. The output may include a prediction of a translation of each point of the encoded 3D model representation into positions corresponding to a crown pose.

Accordingly, blocks of the flowchart 1100 support combinations of means for performing the specified functions and combinations of operations for performing the specified functions. It will also be understood that one or more blocks of the flowchart 1100 can be implemented by special purpose hardware-based computer systems which perform the specified functions, or combinations of special purpose hardware and computer instructions.

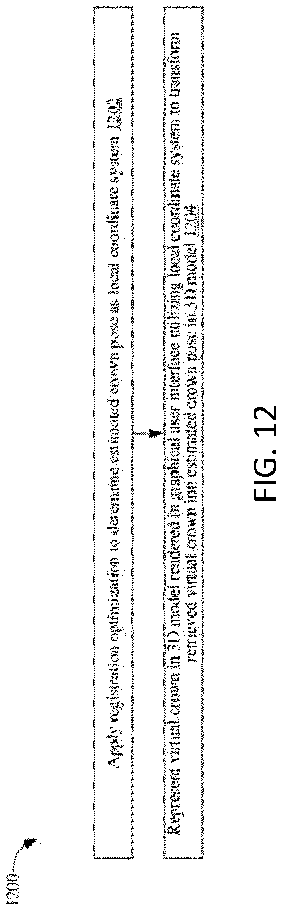

FIG. 12 illustrates a flowchart for processing steps of the encoded 3D model representation, in accordance with an example embodiment. FIG. 12 is explained in conjunction with elements of FIG. 1A, FIG. 1B, FIG. 2, FIG. 3A, FIG. 3B, FIG. 4A, FIG. 4B, FIG. 4C, FIG. 5A, FIG. 5B, FIG.6A, FIG.6B, FIG.7A, FIG.7B, FIG.7C, FIG.8A, FIG.8B, FIG.8C, FIG.8D, FIG. 8D, FIG.8E, FIG.8F, FIG. 9, FIG. 10, and FIG.11. The operations of the exemplary method may be executed by any computing system, for example, by the processors 106 of the system 102 of FIG. 1A. The operations of the flowchart 1200 may start at 1202.

At 1202, a registration optimization may be applied to determine the estimated crown pose as a local coordinate system. The local coordinate system may include an origin, an X-direction pointing along a jaw arch of the dentition, a Y-direction pointing in an occlusal direction and a Z-direction pointing in a buccal direction. Further, the Y-direction is common for each of the one or more teeth in the 3D representation. In an alternative, the Y-direction may be different from each of the one or more teeth in the 3D representation.

At 1204, the virtual crown represented in the 3D model may be rendered in a graphical user interface utilizing the local coordinate system to automatically transform a retrieved virtual crown into the estimated crown pose in the 3D model.

Accordingly, blocks of the flowchart 1200 support combinations of means for performing the specified functions and combinations of operations for performing the specified functions. It will also be understood that one or more blocks of the flowchart 1200 can be implemented by special purpose hardware-based computer systems which perform the specified functions, or combinations of special purpose hardware and computer instructions.

Many modifications and other embodiments of the disclosure set forth herein will come to mind of one skilled in the art to which these disclosures pertain having the benefit of the teachings presented in the foregoing descriptions and the associated drawings. Therefore, it is to be understood that the disclosures are not to be limited to the specific embodiments disclosed and that modifications and other embodiments are intended to be included within the scope of the appended claims. Moreover, although the foregoing descriptions and the associated drawings describe example embodiments in the context of certain example combinations of elements and/or functions, it should be appreciated that different combinations of elements and/or functions may be provided by alternative embodiments without departing from the scope of the appended claims. In this regard, for example, different combinations of elements and/or functions than those explicitly described above are also contemplated as may be set forth in some of the appended claims. Although specific terms are employed herein, they are used in a generic and descriptive sense only and not for purposes of limitation.

## Claims

1. A computer-implemented method (1100) for estimating a crown pose and representing a virtual crown in a 3D model (110) of a dentition (114) of a patient, the method comprising:
obtaining the 3D model (110) of the dentition (114) of the patient, wherein the 3D model (110) represents one or more teeth of the patient;
segmenting the 3D model (110) to obtain segmented tooth data;
generating an encoded 3D model representation suitable for processing by a trained neural network (112), the generating the encoded 3D model representation comprising:
subsampling a point cloud (404) representation based on the segmented tooth data, the point cloud representation comprising a plurality of points representing the one or more teeth of the patient in the 3D model;
determining, for each of the plurality of points, a surface normal representation;
retrieving a dental notation of a restorative site;
encoding each of the plurality of points with the corresponding surface normal representation and with a value relative to the restorative site to produce the encoded 3D model representation;
inputting the encoded 3D model representation into the trained neural network (112);
producing an output, using the trained neural network (112), wherein the output comprises a prediction of a translation of each point of the encoded 3D model representation into positions corresponding to a crown pose, wherein a processing step of the encoded 3D model representation further comprises:
applying a transformation optimization to determine the estimated crown pose as a local coordinate system defining X, Y, Z directions and an origin for placing a virtual crown, and
representing a retrieved virtual crown in the 3D model rendered in a graphical user interface utilizing the local coordinate system by automatically transforming the retrieved virtual crown into the estimated crown pose.

2. The computer-implemented method (1100) according to claim 1, wherein the 3D model (110) comprises a plurality of facets (306) representing the one or more teeth of the patient, and wherein subsampling further comprises generating a point cloud (404) from the plurality of facets (306) representing the one or more teeth.

3. The computer-implemented method (1100) according to claim 1 or 2, wherein the surface normal representation is determined as a surface normal of each of the plurality of facets (306) representing the one or more teeth in the 3D model (110).

4. The computer-implemented method (1100) according to claim 1, wherein the surface normal representation is determined from calculating a point variance of each of the plurality of points of the subsampled point cloud (404).

5. The computer-implemented method (1100) according to claim 1, wherein the retrieved virtual crown is configured as a library virtual crown retrieved from a storage medium or an automated generated crown retrieved from a down-stream processing step.

6. The computer-implemented method (1100) according to claim 1, further comprising:
retrieving regular tooth site notations; and
encoding each of the plurality of points associated with regular teeth at increasing distance from the restorative site dental notation with increasing integer values based on the regular tooth site notations, wherein a positive integer value indicates a first direction from the restorative site and a negative integer value indicates a second direction opposite to the first direction from the restorative site.

7. The computer-implemented method (1100) according to claim 6, wherein the increasing integer values and decreasing integer values are determined by subtracting the dental notation from the regular tooth site notation.

8. The computer-implemented method (1100) according to claim 1, further comprising:
encoding a first set of the plurality of points of the point cloud (404) associated with a first part of the dentition with a first jaw value; and
encoding a second set of points of the plurality of points of the point cloud associated with a second part of the dentition with a second jaw value.

9. The computer-implemented method (1100) according to claim 8, wherein the first jaw value is associated with an upper jaw of the dentition, and the second jaw value is associated with a lower jaw of the dentition.

10. The computer-implemented method (1100) according to claim 1, further comprising encoding each of the plurality of points of the point cloud (404) associated with at least one of: one or more regular teeth, or a restorative site, with a distalness value.

11. The computer-implemented method (1100) according to claim 10, further comprising encoding the distalness value, wherein the distalness value encoding comprises:
one or more first labelling points of teeth associated with a central incisor with a first label.
one or more second labelling points associated with one or more distal teeth sites identifications from the central incisor with consecutive numbering.

12. The computer-implemented method (1100) according to claim 1, further comprising encoding a set of points from the plurality of points associated with a specified tooth with a plurality of different labels, wherein each of the plurality of different labels comprise at least one of: one or more of natural tooth, crown preparation, implant holes, scan body, healing abutment, post preparation, core preparation or other variations of preparation types.

13. The computer-implemented method (1100) according to claim 1, wherein the estimated crown pose comprises a 4 x 4 affine rigid transformation matrix encoding for a global coordinate system to crown pose transformation applied to each of the plurality of points of an input point cloud.

14. A computer-aided dental restoration system (102), comprising:
a scanner device configured to obtain 3D scan data of an intraoral site of a patient;
one or more processors, connected to the scanner device, configured to utilize the 3D scan data to generate a 3D model of the intraoral site of the patient, wherein the one or more processors are configured to carry out a method according to any previous claim 1-13.

15. A computer programmable product comprising a non-transitory computer readable medium having stored thereon computer executable instructions, which when executed by a processing circuitry, cause the processing circuitry to carry out a method according to any previous claim 1-13.
